# EUROPEAN PATENT APPLICATION

(11) **EP 2 596 802 A1**
(43) Date of publication of application: **29.05.2013**
(21) Application number: 11075261.5
(22) Date of filing: 23.11.2011
(51) Int. Cl.: A61K 39/02, A61K 47/34, A61K 9/00

(54) **Pharmaceutical composition for treatment of allergic reactions**

(71) Applicant: PLS-Design GmbH, 20255 Hamburg (DE)
(72) Inventor: Grunwald, Thomas, Dr., 22459 Hamburg (DE); Schmidt-Weber, Carsten B., Prof.Dr., 80802 München (DE)
(74) Representative: Jungblut, Bernhard Jakob

(57) **Abstract**

The invention relates to a pharmaceutical composition made of one or more preparations and comprising a physiologically effective dose of at least one IL-4 and/or IL-13 inhibitor and at least one allergene, and a matrix, wherein at least the inhibitor is solved or embedded, or whereon at least the inhibitor is coated or adsorbed, wherein the matrix is selected as to enable prolonged release of the inhibitor.

## Description

### Field of the invention

The present invention relates to methods for coincident *in vivo* blockade of IL-4- and IL-13-mediated pathways along with allergen stimulation of T cells to augment the induction of clinical tolerance to allergens.

### Background of the invention and state of the art

Allergic diseases including immediate type (type I) hypersensitivity result from an unbalanced response of the specific immune system. In type-I hypersensitivity, Th2 cells upregulate CD40 ligand on their surface upon contact with an allergen-presenting cell. Interaction of CD40 ligand with the CD40 on B cells is accompanied by the production of Th2-related cytokines like IL-4, IL-5 and IL-13 and a subsequent class-switching of the immunoglobulins leads to the production of IgE antibodies which mediate various clinical symptoms.

However, the existence of allergen-specific Th2 cells is not sufficient for the development of allergy since these cells are also found in healthy individuals. Critical for allergy pathogenesis is the number of allergen-specific regulatory T cells (Treg) capable of suppressing the proliferation and cytokine expression of Th1 and Th2 cells, and acting on antigen-presenting cells (APC).

Regulatory T cells are classified broadly as natural T regulatory T cells (nTreg) and induced or adaptive regulatory T cells (iTreg). The natural regulatory T cells are self antigen specific CD4⁺T cells that express FOXP3 transcription factor and high amounts of CD25. They are selected in the thymus and become regulatory T cell in the periphery. Naive CD4⁺Th cells in the periphery can develop into regulatory T cells and, therefore, are called induced or adaptive regulatory T cells. The expression of FOXP3 transcription factor is considered a key factor for the induction of regulatory T cells These cells including type 1 regulatory T cells (Tr1) and Th3 cells can produce IL-10 or TGF-beta or both, by which they exert most of their suppressive activity (for reviews, see Schmidt-Weber, 2005; Nandakumar *et al*., 2009).

The suppression of the immune response by regulatory T cells occurs by various pathways (for reviews, see Schmidt-Weber, 2005; Rouse, 2007; Nandakumar *et al.,* 2009). IL-10 directly and indirectly suppresses the activity of mast cells, basophils and eosinophils. It also promotes IgG4. TGF-beta is a switch and promotion factor for B cells towards IgA isotype and thereby controls peripheral tolerance. Most important, TGF-beta has been shown to promote FOXP3 expression and to inhibit the Th1-driving transcription factor T-BET as well as the Th2-driving transcription factor GATA-3. IL-10 has also been shown to promote FOXP3 expression *in vitro,* but only if IL-4 and IL-12 were neutralized. Both cytokines represent decision signals for T-cell differentiation, and induce the transcription factors GATA-3 or T-BET, respectively.

Generally, Treg-mediated suppression appears to be antigen specific, although the responsible cytokines are secreted and, thereby, potentially are available for a variety of T cells. However, recent studies have shown that cytokines and cytokine receptors can become part of the immunological synapse that is formed between the APCs and the T cells (for a review, see Schmidt-Weber, 2005). Thereby, soluble cytokines can be shuttled in an antigen-specific manner to a neighbouring cell and Tregs can switch a particular target cell into a non-responsive, non-apoptotic state (anergy).

Among the cytokines contributing to the development of allergic inflammation IL-4 and IL-13 play key roles in the pathogenesis of asthma and other allergic diseases. Both cytokines represent pleiotropic cytokines and are associated with the induction of the IgE isotype switch, secretion of IgE by B lymphocytes, and enhancement of IgE-mediated responses by up-regulating IgE receptors on B lymphocytes, mast cells and basophils. However, IL-4 and IL-13 have also some distinct non-overlapping functions. For example, IL-4 is able to drive the differentiation of naive Th cells into Th2 lymphocytes, leading to the production of effector cytokines such as IL-4, IL-5, IL-9, and IL-13. IL-13 has important effects on epithelial cell maturation, mucus production, generation of extracellular matrix proteins, enhancement of the contractility of airway smooth muscle cells, and the recruitment of eosinophils, macrophages and T cells.

IL-4 exerts its activities by interacting with specific Type I and type II receptors on the cell surface (for a review, see Gessner et al., 2000). The type I receptor comprises a 140 kDa binding chain (Swiss Prot accession number P24394), IL-4Rα (also referred to as CD124), and the common γ-chain (γ_{c}) of the IL-2R, which is shared by multiple cytokine receptors. In the absence of the common γ-chain IL-4 can use the type II IL-4 receptor, comprising IL-4Rα and an IL-13 binding chain, IL-13Rα1. This receptor complex is also a functional receptor for IL-13, and this explains the overlap of the majority of biological effects of IL-4 and IL-13, although both cytokines exhibit only 25% homology. Type I receptor complexes can only be formed by IL-4 and appear to be responsible for mediating IL-4 responses in T cells which do not express IL-13Rα1. Type II receptor complexes can be formed and activated by either IL-4 or IL-13. The type I receptor complex predominates in hematopoietic cells, whereas the type II receptor complex is expressed on both hematopoietic cells and non-hematopoietic cells. The binding sequence of IL-4 and IL-13 to type II receptor complexes differs in that IL-4 first binds to IL-4Rα with high affinity before associating with the second subunit, whereas IL-13 first binds to IL-13Rα1 with low affinity and then this complex recruits IL-4Rα to form a high affinity binding site.

A second IL-13 binding protein, IL-13 receptor α2 (IL-13Rα2), binds IL-13 with high affinity but does not bind IL-4. However, IL-13Rα2 is believed to be non-signaling, thereby serving as a decoy receptor. It has been shown that treatment of epithelial cells with IFN-γ increases surface expression of IL-13Rα2 through mobilization from intracellular stores, suggesting that IL-13Rα2 plays a key role in responding to and regulating the Th1/Th2 balance by competing with the IL-4Rα/ IL-13Rα1 complex for IL-13.

Soluble IL-4 receptors (sIL-4Rα) lacking the transmembrane and intracellular domains are present in biological fluids. While the precise function of sIL-4Rα remains to be elucidated, it may even enhance IL-13 responses by forming a complex with IL-13Rα1 on the cell surface, thereby stabilizing the weak interaction of IL-13 with IL-13Rα1. When tested in bronchial fibroblasts, the presence of sIL-4Rα enhanced IL-13-stimulated eotaxin production in response to suboptimal doses of IL-13. In addition, sIL-4Rα may act as a carrier protein from which IL-4 can dissociate over time. Therey, sIL-4Rα could offer some kind of protection of IL-4 against proteolytic degradation.

### Specific immunotherapy

Specific allergen immunotherapy (IT) is the only widely used treatment capable of restoring clinical tolerance to allergen and is associated with the re-induction of allergen-specific Treg cells. Peripheral tolerance to allergen in the normal immune response of healthy individuals to allergen exposure and in specific immunotherapy is mainly the result of sufficient Treg numbers. Therefore, the main principle of specific immunotherapy in atopic patients is to increase the number of Tregs capable of controlling allergen-specific effector T cells.

Successful IT is associated with a change of T cell memory from the pro-allergic Th2 phenotype towards a peripheral IL-10-producing T cell phenotype and a local phenotype that also shows FOXP3 expression in addition to IL-10 positive cells. It was previously shown that immunotherapy can result in long-term benefit with disease remission for 3 years following discontinuation. However, considering the various factors influencing the efficacy of specific immunotherapeutic approaches, it is not surprising that the clinical outcome varies to a great extent. Immunotherapies are efficient when patients are monosensibilized against seasonal allergens, but can be less or not efficient if the patient is atopic or if the patient reacts to perennial allergens.

One problem appears to be the quantity of individual allergens in allergen extracts derived from natural sources since in some extracts important allergens are not present in sufficient amounts. However, successful specific immunotherapy requires application of high allergen doses which promote the engagement of the low-affinity FcεRII (CD23) known as a negative feedback regulator of IgE-dependent responses. If present in sufficient quantities the administered allergen will not only be bound by specific IgE, but also by other immunoglobulin classes. IgG complexes with allergen are important in modulating the immune response and preventing IgE-mediated reactions. The existence of three types of Fcγ-receptors is known. FcγRI und FcγRIII, expressed primarily on cells of the myeloid lineage, can mediate pro-inflammatory functions such as phagocytosis, antibody-dependent cytotoxicity and the release of inflammatory mediators. FcγRII expressed on both myeloid and lymphoid cells, transmits inhibitory signals into the cells. Activated dendritic cells are potent T-cell stimulators and express both inhibitory and activatory FcγRs. In view of these facts the recent development or recombinant allergens for specific immunotherapy appears to be a promising approach since they allow the design of allergen compositions containing all important allergens in optimal concentrations. In addition, recombinant allergens allow site-directed modifications of their surface structure in order to decrease the density of B cell epitopes. The aim of such allergen modification is to decrease the allergenicity while retaining its immunogenicity. Evaluation of modified recombinant allergens with a strongly reduced IgE reactivity that display the full spectrum of linear T cell epitopes but a different surface structure as compared to the corresponding natural allergen, have demonstrated that such molecules are capable of reducing specific IgE development towards the native allergen (Niederberger *et al*., 2004; Karamloo *et al*., 2005).

However, despite recent improvements the efficacy of specific immunotherapy needs to be further optimized. Appropriate approaches for increasing the number of Tregs capable of controlling allergen-specific effector T cells remain to be defined. Therefore, there is a need in art to define critical factors influencing the induction of Tregs and to design more effective allergen-tolerogenic therapies.

### Technical Problem of the invention

Thus, the technical problem of the invention is to provide means for treatment of allergic reactions, wherein the induction of clinical tolerance to allergenes is improved, in particular wherein the systemic side effects of IL-3 and/or IL-13 inhibitor administration are reduced and in combination therewith the stimulation of T-cells for balancing the resonse of the specific immune system is enhanced. Specifically, the technical problem is to provide a pharmaceutical composition, uses thereof, a method for making such composition and a treatment method for achieving these goals.

### Summary of the invention

For solving these technical problems the invention provides the subject matters defined in the claims. The invention is explained in more detail in the following.

Peripheral tolerance to allergen in the normal immune response of healthy individuals to allergen exposure and in specific immunotherapy is mainly the result of sufficient Treg numbers. Therefore, the main principle of specific immunotherapy in atopic patients is to increase the number of Tregs capable of controlling allergen-specific effector T cells. Under *in vitro* conditions, the allergen and the absence of Th1- or Th2-driving factors have been demonstrated to be essential for the induction of Tregs. Since IL-4 and IL-13 play key roles in the development of allergic inflammation, the present invention provides methods for coincident *in vivo* inhibition of IL-4-and IL-13-mediated effectsd along with allergen stimulation of T cells to augment the induction of clinical tolerance to allergens.

Preferred inhibitors of IL-4- and IL-13-mediated effects suitable for the method of the present invention include but are not limited to a) antagonistic IL-4 and IL-13 derivatives, b) soluble Il-4 and IL-13 receptor constructs, c) monoclonal antibodies, fragments thereof or other proteinaceous constructs with specificity for IL-4 and IL-13 capable of blocking their biologic activity, d) monoclonal antibodies, fragments thereof or other proteinaceous constructs with specificity for IL-4 and IL-13 receptor complexes capable of blocking the interaction of IL-4 and IL-13 with their receptor subunits, e) aptamers with specificity for IL-4 and IL-13 capable of blocking their biologic activity, and f) aptamers with specificity for IL-4 and IL-13 receptor complexes capable of blocking the interaction of IL-4 and IL-13 with their receptor subunits.

In one embodiment, the present invention discloses methods for a prolonged inhibition of IL-4- and IL-13-mediated effects at the site of allergen presentation along with a prolonged presentation of allergens. To achieve effective and prolonged inhibition of IL-4- and IL-13-mediated effects at the site of allergen presentation, the present invention discloses methods for a sustained release of such inhibitors or combinations of such inhibitors from a depot at the site of allergen presentation.

In a specific embodiment, the present invention discloses matrices for prolonged delivery of inhibitors of decision signals for T-cell differentiation. Preferred matrices include but are not limited to biodegradable polymers which are suitable as depot for substantial quantities of such inhibitors or inhibitor combinations, which allow the release of sufficient quantities of the inhibitor or inhibitor combinations for efficient local inhibition of IL-4- and IL-13-mediated pathways over a prolonged period of time, and which are chemically and physically compatible with the adjuvant and allergen (s) used for the induction of tolerance according to the method of the present invention.

In a preferred specific embodiment, injectable in situ-forming gel systems which are biodegradable, are used for controlled delivery of inhibitors of IL-4- and IL-13-mediated pathways. Such in situ-forming gel systems (hydrogels) undergo a sol-gel-sol transition, which is free flowing sol at room temperature and a non-flowing gel at body temperature. Compared to other biodegradable polymers, the injectable thermogelling polymers possess several advantages including easy preparation, high encapsulation efficiency of bioactive molecules such as inhibitors of IL-4- and IL-13-mediated pathways, and free of harmful organic solvents in the formulation process.

In another embodiment, the present invention discloses methods for restricting high local concentrations of inhibitors of IL-4- and IL-13-mediated effects mainly to the site of allergen presentation to reduce adverse effects due to interaction of the inhibitors with targets distal from the site of allergen presentation. In one specific embodiment, inhibitors of IL-4- and IL-13-mediated effects are used which provide a relatively short serum half-life that is sufficient for the inhibitors to be locally active upon their release from a depot at the site of allergen presentation, and which allows fast removal from circulation upon diffusion and transport away from the site of allergen presentation. Preferred inhibitors providing such characteristics include but are not limited to a) antagonistic IL-4 and IL-13 derivatives with a molecular weight of ≤15 kDa, b) small antibody fragments or other proteinaceous constructs capable of inhibiting IL-4- and IL-13-mediated effects, and c) small molecular weight aptamers capable of inhibiting IL-4- and IL-13-mediated effects. In another specific embodiment, inhibitors of IL-4- and IL-13-mediated effects are used which are susceptible to inactivation by endogeneous enzymes after their release from a depot at the site of allergen presentation and which can be derivatized to adjust their susceptibility to enzymatic inactivation according to the needs of the method of the present invention. Preferred inhibitors providing such characteristics include but are not limited to aptamers the susceptibility of which to degradation by endogenous nucleases can be adjusted by chemical modification of the bases and the backbone structure.

In another embodiment, the present invention discloses adjuvants suitable for the method of the present invention. In a preferred embodiment, adjuvants are used which provide a depot effect for the allergens to be adminstered and which elicit Th2-type immune responses. Although adjuvants elicting primarily a Th2-type immune response are potentially interfering with the induction of Tregs, in the presence of inhibitors of Il-4- and IL-13-mediated pathways such adjuvants are better suited for the therapeutic aims of the present invention than those elicting primarily a Th1-type immune response. Preferred adjuvants eliciting Th2-type immune responses include but are not limited to aluminum salts, Montanide emulsions (squalene-based water-in-oil emulsions) and polyphosphazenes. Adjuvants eliciting a combined Th1- and Th2-type immune resonse may also be used for the method of the present invention if such adjuvants provide advantageous properties for the composition comprising the allergen(s), an adjuvant, inhibitors of IL-4- and IL-13-mediated pathways, and a matrix mediating prolonged delivery of such inhibitors. Adjuvants eliciting a combined Th1-type and Th2-type immune response include but are not limited to squalene-based oil-in-water emulsions, granulocyte-macrophage colony stimulating factor (GM-CSF), and adjuvants based on inulin and virosomes.

In another embodiment, the present invention discloses vaccine compositions which are useful for the method of the present invention. In one specific embodiment, the vaccine composition comprises a biodegradable thermogelling polymer solution containing one or more soluble inhibitors of IL-4-and IL-13-mediated pathways. Upon injection this fluid formulation then spontaneously gels as the temperature of the formulation rises to body temperature. Thereby, the injected gel forms a non-flowing depot from which one or more soluble inhibitors of IL-4- and IL-13-mediated pathway are released slowly and continuously for several days. The quantity of inhibitors of IL-4- and IL-13-mediated pathways in the composition is balanced in a way that upon gellation of the polymer composit at body temperature the amount of released inhibitors is sufficient for the therapeutic aims of the method of the present invention. For prolonged allergen presentation at the site of the gelled polymer composit, one or more allergens or an allergen extract are adsorbed onto aluminium salts or emulsified to form a squalene-based water-in-oil emulsion (Montanide emulsion), and injected in close proximity of the gelled polymer composit.

In another specific embodiment, the vaccine composition comprises a biodegradable thermogelling polymer solution containing one or more soluble inhibitors of IL-4- and IL-13-mediated pathway and one or more allergens (or an allergen extract) either adsorbed onto aluminium salts, or emulsified to form a squalene-based water-in-oil emulsion (Montanide emulsion), or premixed with a water-soluble polyphosphazenes polymer adjuvant. The quantity of each component in the composition is balanced in a way that a) upon injection into the body the biodegradable thermogelling polymer forms a non-flowing gel in which the other components are embedded, and b) upon gellation of the polymer composit at body temperature the amount of released components is sufficient for the therapeutic aims of the method of the present invention.

In still another specific embodiment, the vaccine composition comprises a biodegradable thermogelling polymer solution containing one or more allergens (or an allergen extract), one or more soluble adjuvants (such as GM-CSF), and one or more soluble inhibitors of IL-4- and IL-13-mediated pathways. The quantity of each component in the composition is balanced in a way that a) upon injection into the body the biodegradable thermogelling polymer forms a non-flowing gel in which the other components are embedded, and b) upon gellation of the polymer composit at body temperature the amount of released components is sufficient for the therapeutic aims of the method of the present invention.

In another embodiment, the present invention discloses methods for incorporating vaccine compositions into pharmaceutical compositions suitable for administration.

In yet another embodiment, the present invention discloses therapeutic methods for the induction of allergen tolerance using the vaccine compositions of the present invention.

Specific preferred embodiments of the present invention will become evident from the following more detailed description and the claims.

### Detailed description of the invention

Based on current knowledge, allergen tolerance is mediated by peripherally induced regulatory T cells as evidenced by a deficit of allergen-specific IL-10 producing T cells in the peripheral blood of allergic patients (for reviews, see Schmidt-Weber *et al.,* 2005; Schmidt-Weber *et al.,* 2006). The main principle of specific immunotherapy in atopic patients is to increase the number of Tregs capable of controlling allergen-specific effector T cells. The expression of FOXP3 transcription factor is considered a key factor for the induction of regulatory T cells. We and others have shown that TCR receptor triggering is essential to induce FOXP3 expression and suppressive T cells in humans (Schubert *et al*., 2001; Mantel *et al*., 2006). We demonstrated that GATA3 induced by IL-4 inhibits the expression of FOXP3, which is a requirement for inducible Treg (iTreg) differentiation (Mantel *et al*., 2007). We have demonstrated this functional antagonism *in vivo* and in vitro and have established that the mechanistic basis for this is IL-4-dependent binding of GATA3 to the FOXP3 promoter with subsequent inhibition of FOXP3 transcription. As a consequence this antagonism torpedoes induction of Tregs and tolerance in conditions that are associated with excessive IL-4 production.

Under *in vitro* conditions, the antigen and the absence of Th1- or Th2-driving factors have been demonstrated to be essential for the induction of Tregs. Based on this observation it has been postulated that the generation of Tregs represents a default pathway that requires T-cell receptor activation as for any other T-cell differentiation, but the absence of decision signals for effector T cells. It is known, however, that IL-4 is released locally upon allergen injection due to the activation of Th2 memory T cells and IL-4 will also be released during the vaccination process. Therefore, in the present invention methods have been designed for coincident *in vivo* blockade of IL-4-mediated pathways along with allergen stimulation of T cells to augment the induction of clinical tolerance to allergen.

Since IL-13 is also known to be associated with the induction of the IgE isotype switch, secretion of IgE by B lymphocytes, and enhancement of IgE-mediated responses, methods of the present invention include also coincident *in vivo* blockade of IL-13-mediated pathways along with allergen stimulation of T cells. Although T cells lack an IL-13 binding receptor component and do not respond to IL-13, several studies indicate that IL-13 blockade could further augment the induction of clinical tolerance to allergen.

Most important, the present invention discloses methods for a prolonged inhibition of IL-4- and IL-13-mediated effects along with a prolonged presentation of the allergens. Prolongation of the process is essential since T cell differentiation and thus the development of immunologic memory requires the engagement of the T cell receptor (TCR) over 12-48 h and long lasting memory may even require repetitive exposure. Accordingly, inhibition of IL-4- and IL-13-mediated effects needs to be in effect at least for the same period of time, most likely as long as allergens are released from the injected allergen-adjuvant complex. Otherwise, released allergen molecules may induce Th2-driving factors, thereby inhibiting the induction of Tregs. The present invention discloses methods for a steady release of inhibitors of IL-4-and IL-13-mediated effects from a depot at the site of allergen presentation.

The present invention discloses also methods for establishing high local concentrations of inhibitors of IL-4-and IL-13-mediated effects at the site of allergen presentation which is as important as a prolonged presence of inhibitors. Since IL-4 can be shuttled in an antigen-specific manner in the immunological synapse that is formed between the antigen presenting cells and the T cells, effective inhibition of IL-4-mediated effects in the micro-environment of the immunological synapse requires a high local inhibitor concentration. Although T cells do not express IL-13Rα1, type II receptor complexes mediating IL-13-induced effects are expressed on both hematopoietic cells and non-hematopoietic cells in close proximity of the immunological synapse that is formed between the antigen presenting cells and the T cells. Therefore, effective inhibition of IL-13-mediated effects can be assumed to require also a high local inhibitor concentration.

High local concentrations of inhibitors, however, are extremely difficult to achieve via systemic administration and high doses of therapeutics are frequently associated with increased adverse effects. For example, the IL-4 mutein IL-4/Y124D (substitution of tyrosine with aspartic acid at position 124) binds to IL-4Rα with the same affinity as the wild-type IL-4, but has only 0.5% of the agonistic activity (Letzelter *et al*., 1998). This IL-4 mutein is a potent inhibitor of IL-4-mediated effects, but in vivo experiments have demonstrated that IL-4/Y124D exhibits acute toxicity upon systemic application similar to that of wild-type IL-4 in monkeys. The cellular events associated with both wild-type IL-4- and IL-4/Y124D-mediated toxicity include upregulation of VCAM-1 (vascular cell adhesion molecule 1), upregulation of MCP-1 (monocyte chemotactic protein 1) in serum, increases in circulating monocytes together with a concomitant decrease in circulating lymphocytes, and an increase in hematocrit (WO 97/47744). Similar cellular trafficking upon systemic application has been observed in clinical trials using IL-4 in humans (Wong *et al*., 1992). These results suggest that these toxicities of IL-4/Y124D are due to agonist activities mediated through cells other than T cells. Based on the known effects of Il-4 on endothelial cells, the observed toxicities of IL-4/Y124D point to interactions of this IL-4 mutein with a novel IL-4 receptor complex expressed on endothelial cells (WO 97/47744). This example demonstrates the necessity for restricting high local concentrations of inhibitors of IL-4-and IL-13-mediated effects mainly to the site of allergen presentation.

The method of the present invention solves this problem by disclosing procedures which utilize inhibitors of IL-4- and IL-13-mediated effects with a relatively short half-life. Such inhibitors are locally active upon their release from a depot at the site of allergen presentation and are quickly removed from circulation upon diffusion and transport away from the site of allergen presentation. For example, US Patents 6,028,176 and 6,313,272 disclose an IL-4 mutein with potent antagonistic activity but a relatively short plasma half-life in vivo of approximately 3 to 6 hours.

In consequence, the present invention discloses methods that provide a high local concentration of inhibitors for a prolonged period of time at the site of allergen presentation associated with a limited radius of inhibitory activity away from the site of allergen presentation due to rapid clearance. As a result, potential adverse effects mediated by the inhibitors are reduced significantly.

I. *Inhibitors of decision signals for T-cell differentiation* For the present invention several principles for local inhibition of the effects of IL-4 and IL-13 are suitable (for a review, see Long, 2009). Preferred principles include but are not limited to the application of antagonistic cytokine derivatives, soluble cytokine receptor constructs, monoclonal antibodies, fragments thereof or other proteinaceous constructs with specificity for IL-4 and Il-13 or their respective receptors, and anti-cytokine and anti-cytokine receptor aptamers. Any compound that functions as an IL-4, IL-13 or IL-4/IL-13 antagonist and is suitable for administration in accordance with the method of the present invention may be employed. Antagonists do not need to completely abolish IL-4-or IL-13-induced biological activity to be useful. Rather, a given antagonist may reduce a biological activity of IL-4 and/or IL-13. Combinations of two or more antagonists may be employed in methods and compositions of the present invention.

Most preferred inhibitors of IL-4 and IL-13 are those which a) provide a small to moderate molecular size and high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation.

The vast majority of the anti-IL-4 and anti-IL-13 principles which are useful for the therapeutic aims of this invention, have been published and evaluated in animal models as well as in clinical trials for the treatment of asthma (for a review, see Long, 2009). However, application of these inhibitors according to the method of the present invention has not been disclosed.

Many inhibitors of IL-4- and IL-13-mediated pathways including antagonistic cytokine derivatives, soluble cytokine receptor constructs, anti-cytokine and anti-cytokine receptor antibodies have been developed to interrupt or to reduce Th2 dependent allergic inflammation in patients with asthma. The therapeutic aim is a shift of the patient's immune response towards a cytotoxic T-cell-mediated immune response, e.g., characterized by a Th1-type immune phenotype. In contrast, the therapeutic aim of the present invention is not a shift from a Th2-type immune phenotype to a Th1-type immune phenptype, but the induction of Tregs in a local micro-environment that is free of Th1- or Th2-driving factors despite the presence of allergens and adjuvants elicting a Th2-type immune response.

In addition to applications for treatment of asthma, patent WO 2009/081 201 A2 discloses clinical applications of an IL-4Rα inhibitor for use a) in allergy therapy to suppress IgE synthesis (including for example atopic dermatitis and food allergy), b) for transplantation therapy to prevent transplant rejection, and c) for suppression of delayed-type hypersensitivity or contact hypersensitivity reactions. However, details of the suggested clinical applications are not disclosed.

In general, the rationale in published applications of IL-4 inhibition is a shift of the patient's Th2-type immune response towards a Th1-type immune response. For example, the application of inhibitors of IL-4-mediated pathways for the treatment of cancer is based on the assumption that it is sometimes beneficial for the patient to promote a Th1-type immune response (WO 02/04009 A2). Furthermore, US Patent No 2011/0002013 A1 discloses that IL-4 antagonists find use as adjuvants to allergy immunotherapy and as vaccine adjuvants, especially when directing the immune response towards a Th1 response would be beneficial in treating or preventing the disease in question. Again, details of the suggested application of IL-4 antagonists as adjuvants to allergy immunotherapy are not disclosed.

### II. Inhibition of decision signals for T-cell differentiation by interleukin variants

In one embodiment of the invention, interleukin variants capable of antagonizing the activity of IL-4 and IL-13 are used as inhibitors. Any interleukin variant that functions as an IL-4 , IL-13 or IL-4/IL-13 antagonist and is suitable for administration in accordance with the method of the present invention may be employed. Interleukin-based antagonists do not need to completely abolish IL-4- or IL-13-induced biological activity to be useful. Rather, a given antagonist may reduce a biological activity of IL-4 and/or IL-13.

Antagonistic IL-4 mutants have been described (Kruse *et al*., 1992; Muller et al., 1994; US patent 6,028,176; US patent 5,723,118). Within the four helix bundle of human IL-4, tyrosine (Y124) on the D helix forms an important contact with the common γ-chain (γ_{c}). When Y124 is mutated to aspartic acid (Y124D), the resulting molecule binds to IL-4Rα with the same affinity as the wild-type IL-4, but has only 0.5% of the agonistic activity (Letzelter *et al*., 1998). Similarly, the arginine at position 121 of human IL-4 interacts with IL-13Rα1. Mutations of R121 generates mutants that can bind to IL-4Rα, but are antagonists of both IL-4- and IL-13-induced responses (Kruse *et al*., 1993). However, other or additional mutations of IL-4 may also be considered for generating IL-4 variants suitable for the method of the present invention. For example, by mutation of serine at position 125 of IL-4 mutants have been generated that can bind to IL-4Rα, but are antagonists of both IL-4- and IL-13-induced responses (Kruse *et al*., 1993). Suitable for the method of the present invention are antagonistic interleukin-4 variants with single, double and triple mutations, single mutations including but not limited to amino acid positions R121, Y124, and S125; double and triple mutations including but not limited to combinations of the above listed amino acid positions. Preferred are antagonistic IL-4 variants with a relatively short half-life to limit adverse effects distant from the site of allergen presentation.

The double mutant of human IL-4 (R121D/Y124D) has been shown to protect allergic cynomolgus monkeys from allergen-induced airways hyper-responsiveness when administered either subcutaneously or via nebulisation. Furthermore, subcutaneous administration of this double mutant in monkeys for 6 weeks or more also reduced the cutaneous wheal response and circulating concentrations of allergen-specific IgE. The effect of the double mutant of IL-4 (R121D/Y124D) on late phase asthmatic response to allergen challenge in asthmatic patients has been evaluated recently in clinical studies (Wenzel *et al*., 2007). The studies demonstrate that dual inhibition of IL-4 and Il-13 can positively affect the course of late asthmatic response after experimental allergen challenge. Treatment with the double mutant was associated with few adverse events, whether administered by subcutaneous injection (up to 30 mg for up to 13 weeks) or by inhalation (up to 60 mg for up to 4 weeks) in participants with atopic asthma or atopic eczema. However, it should be stressed that application of this double mutant for allergy immunotherapy according to the method of the present invention has not been disclosed.

More than 10 years ago animal experiments have demonstrated that inhibition of the IL-4/Il-13 receptor system can completely abrogate humoral immune response to allergen and development of allergic symptoms *in vivo* (Grunewald *et al*., 1999). Treatment of BALB/c mice with the murine IL-4 mutant Q116D/Y119D (the murine equivalent of the human IL-4 double mutant R121D/Y124D) before and after immunization with ovalbumin (OVA) completely inhibited synthesis of OVA-specific IgE and IgG1. In addition, the synthesis of specific IgG2a, IgG2b and IgG3 was suppressed, which may indicate the development of tolerance toward OVA. Immunization was performed by i.p. injection of 10 µg OVA in the presence of Alum as an adjuvant. At day 0, the murine IL-4 mutant was applied by i.p. injection 2 hours pre- and post-OVA-immunization as a 50 µg dose each. From day 1 to 8 treatment was continued with 30 µg mutant twice a day by i.p. injection. The authors concluded that the murine IL-4 mutant Q116D/Y119D inhibits antigen-specific humoral immune responses and allergic symptoms mediated either by IgE or IgG1 *in vivo* and, therefore, should be advantageous for therapy of atopic disorders and other Th2-dominated diseases. Although the results of this study are close to the method and the therapeutic aim of the present invention, the study of Grunewald *et al*. (1999) does not disclose how to provide a high local concentration of inhibitors for a prolonged period of time at the site of allergen presentation associated with a limited radius of inhibitory activity away from the site of allergen presentation due to rapid clearance. Grunewald *et al*. (1999) report the apparent induction of tolerance to the OVA allergen by starting the application of the murine IL-4 mutant Q116D/Y119D before immunization with OVA, which is not comparable to immunotherapy of patients with an existing allergy. For the induction of tolerance to allergens in individuals with a Th2-type immune phenotype only the method of the present inventions provides appropriate means to ensure a sufficient induction of Tregs in a local micro-environment that is free of Th1- or Th2-driving factors despite the presence of allergens.

IL-13 mutants useful for the method of the present invention include but are not limited to the IL-13 mutant E13K which is a powerful antagonist of human IL-13 and capable of partially inhibiting the responses of human IL-4 as well (Kioi et al., 2004). Glutamate at position 13 in IL-13 associates with IL-4Rα and a mutation to lysine decreases its binding to IL-4Rα. Although IL-13E13K prevents binding of IL-4Rα to IL-13Rα1, full inhibition of IL-4 responses cannot be achieved due to the availability of the alternative IL-4R complex, IL-4Rα:γ_{c}.

In a preferred embodiment the human IL-4 double mutant R121D/Y124D is used for inhibition of IL-4- and IL-13-mediated pathways in accordance with the method of the present invention. The human IL-4 double mutant R121D/Y124D is able to antagonize the activity of IL-4 as well as that of IL-13 (Grunewald *et al*., 1998; Tony *et al*., 1994).

### III. Inhibition of decision signals for T-cell differentiation by soluble cytokine receptor constructs

In one embodiment of the invention, soluble cytokine receptor constructs are used for the method of the present invention to inhibit IL-4- and IL-13-mediated pathways. Preferred constructs include but are not limited to the soluble extracellular domain of IL-4Rα, variants, fragments and derivatives thereof that retain the ability to bind IL-4. Human as well as murine IL-4 receptors and their nucleotide sequences have been described (US patent 5,599,905; Idzerda *et al*., 1990 (human IL-4 receptor); Mosley *et al.,* 1989 (murine IL-4 receptor)). The encoded human IL-4 receptor comprises an N-terminal signal peptide, followed by an extracellular domain, a transmembrane region corresponding to amino acids 208 through 231, and a cytoplasmic domain corresponding to amino acids 232 through 800. IL-4 receptor polypeptides arising from alternative mRNA constructs, e.g. which can be attributed to different mRNA splicing events following transcription, and which yield polypeptides capable of binding IL-4, are among the IL-4 receptor polypeptides disclosed herein. IL-4 receptor variants include those having amino acid or nucleotide acid sequences that vary from a native sequence by one or more substitutions, deletions, or additions, but retain a biological activity of the IL-4 receptor protein. The present invention also includes IL-4 receptor proteins with or without associated native-pattern glycosylation. The glycosylation pattern may vary according to the type of host cells in which the protein is produced. Another option is inactivation of N-glycosylation sites by site-directed mutagenesis. Derivatives of the IL-4 receptor protein include those exibiting a different structure as compared to the native protein, having derivatized amino acids (e.g., by cross-linking reagents, or polyethylene glycol).

In another embodiment, the soluble extracellular domain of IL-4Rα, variants, fragments and derivatives thereof that retain the ability to bind IL-4, are conjugated chemically or fused to the N-terminus or C-terminus of other proteins or polypeptides. Preferred IL-4 receptor fusion proteins or polypeptides include but are not limited to peptides facilitating purification (e.g.,His-tag) or identification (e.g., Flag peptide), and proteins or peptides that have the property of promoting oligomerization (e.g., leucine zippers). In a preferred specific embodiment, the soluble extracellular domain of IL-4Rα is fused to the Fc portion or selected constant domains of a human immunoglobulin of any isotype, or a fragment thereof capable of dimerization. If fusion proteins are made with both heavy and light chains of an antibody, it is possible to form an oligomer with as many as four extracellular regions of the IL-4 receptor. Preferred constructs include also hetero-oligomers in which both the soluble extracellular domain of IL-4Rα and the soluble extracellular domain of IL-13Rα1 are fused to a human protein or polypeptide capable of oligomerization.

Among cytokine receptor-based inhibitors suitable for the method of the present invention heterodimers containing both the soluble extracellular domain of IL-4Rα and the soluble extracellular domain of IL-13Rα1 are most preferred. The solubilized IL-4 receptor fragment consisting of the extracellular portion of the human IL-4R-α chain (shIL-4R) that competitively inhibits the binding of IL-4 to its receptor, appeared promising in early human studies (Borish *et al*., 2001). However, a subsequent large scale clinical trial indicated that this reagent had no clinical efficacy in asthma. This poor response could be a consequence of complex formation of shIL-4R with IL-13Rα1 on the cell surface, thereby stabilizing the weak interaction of IL-13 with IL-13Rα1 leading to enhanced IL-13 responses. In order to inhibit both IL-4- and IL-13-mediated responses, a soluble heterodimeric receptor construct has been generated which contains the extracellular domains of IL-4Rα and IL-13Rα1, both fused to the Fc portion of human IgG1 (Economides *et al*., 2003). This heterodimeric construct binds IL-4 and IL-13 with high affinity. IL-13 binds to non-complexed IL-13Rα1 with low affinity, but complexes of IL-13Rα1 and IL-4Rα form a high affinity binding site for IL-13 and IL-4 binds even to non-complexed IL-4Rα with high affinity (Andrews et al., 2006). Application of a heterodimeric construct providing binding sites for IL-13 and IL-4 for allergy immunotherapy according to the method of the present invention has not been disclosed.

### IV. Inhibition of decision signals for T-cell differentiation by antibodies

In another embodiment, antibodies that function as IL-4 or IL-13 antagonists are employed for the method of the present invention. The antibodies preferably are monoclonal antibodies or antigen-binding fragments thereof. Advantageously, humanized or chimeric monoclonal antibodies are employed. Most preferred are human monoclonal antibodies. Examples of suitable antibodies are those that interact with the binding of IL-4 or IL-13 to an IL-4 receptor or Il-13 receptor, respectively. Such antibodies, referred to herein as blocking antibodies, may be raised against either the cytokines IL-4 and IL-13 or the cytokine receptors IL-4Rα and IL-13Rα1, and screened in conventional assays for the ability to interfere with binding of IL-4 and IL-13 with their respective receptor subunits. Antigen-binding fragments of such antibodies including Fab and F(ab')2 or artificial antibody constructs such as scFv or other proteinaceous constructs such as anticalins are also suitable for the method of the present invention. Additional embodiments include chimeric antibodies and antigen-binding fragments thereof, e.g., humanized versions of murine monoclonal antibodies, and human monoclonal antibodies as well as antigen-binding fragments thereof.

In a preferred embodiment, combinations of two or more humanized or human monoclonal antibodies with specificity for IL-4 and IL-13 which are capable of blocking the biologic activity of both cytokines, are used for the method of the present invention. In accordance with the method of the present invention, the biologic activity of both cytokines needs to be blocked to create a local micro-environment at the site of allergen presentation that is free of Th1- or Th2-driving factors.

Suitable monoclonal antibodies capable of blocking the biologic activity of IL-4 and IL-13 have been reviewed recently (Holgate *et al.,* 2008; Long, 2009). For example, the anti-IL-4 humanized monoclonal antibody pascolizumab has been shown to effectively block IL-4 responses *in vitro* (Hart *et al*., 2002). Although this antibody yielded unimpressive results in the treatment of asthma, it may be a promising candidate for the method of the present invention. Binding of IL-13 to IL-4Rα can be blocked effectively with the fully humanized monoclonal IgG1 antibody IMA-638 and the interaction of IL-13 with IL-13Rα1 can be inhibited with the fully humanized monoclonal IgG1 antibody IMA-026 (Gauvreau *et al*., 2010). Both antibodies have shown efficacy in the treatment of asthma in cynomolgus monkeys, but only IMA-638 was able to reduce both early and late phase asthmatic responses in a clinical Phase II study (Gauvreau *et al.,* 2010). Application of such antibodies, fragments and artificial constructs thereof, as well as other anti-cytokine proteinaceous constructs for allergy immunotherapy according to the method of the present invention has not been disclosed.

In another embodiment of the invention, antibodies capable of inactivating cytokine receptors are used to inhibit IL-4- and IL-13-mediated pathways. Preferred are humanized or human monoclonal antibodies with specificity for IL-4Rα which are capable of blocking the biologic activity of both IL-4 and IL-13. For example, the fully human monoclonal IgG2 antibody AMG317 binds with high affinity (K_{d} = 1.8 x 10⁻¹⁰ M) to IL-4Rα and potently blocks the biologic activities of both IL-4 and IL-13 (Corren *et al*., 2010). Furthermore, AMG317 has been shown to have important effects on inflammation in vitro. Although in a Phase II study in patients with asthma AMG317 did not demonstrate clinical efficacy across the overall group of patients (Corren *et al.,* 2010), this antibody provides preferred properties for the method of the present invention. However, it should be stressed that application of this antibody and other anti-cytokine receptor antibodies, fragments and artificial constructs thereof, as well as other anti-cytokine receptor proteinaceous constructs for allergy immunotherapy according to the method of the present invention has not been disclosed.

### V. Inhibition of decision signals for T-cell differentiation by aptamers

In another embodiment of the invention, aptamers are used to inhibit IL-4- and IL-13-mediated pathways. Aptamers are nucleic acid binding species generated by iterative rounds of in vitro selection (for reviews, see Famulok *et al.,* 1999; Yan *et al.,* 2005). Typically, selected aptamers bind very tightly (often in the low nanomolar range) and specifically to their targets. Aptamers can also discriminate between closely related protein targets. Aptamers have several key features that make them particularly well suited as reagents for the method of the present invention. First, aptamers are relatively small and can readily access sites which are difficult to target with large molecules such as the immunological synapse that is formed between the antigen presenting cells and the T cells. Another advantage for the present invention is the possibility to engineer the stability of aptamers towards degrading nucleases, thereby determining the period of their inhibitory activity. Unmodified RNA- and DNA-based aptamers can begin degrading in serum within minutes, whereas aptamers containing modified bases and phosphorothioate linkages display a significantly increased stability towards degrading nucleases. For example, 2'-aminopyrimidine-modified RNA has been shown to remain stable for days. Furthermore, modifications can also be applied to the ends of aptamers to confer greater stability. For example, a 3'-3' linkage can be added to prevent 3'-exonuclease degradation. Stable aptamers can be generated by the Spiegelmer-technology (for a review, see Famulok *et al*., 1999) or by utilizing peptide nucleic acids (EP 1 785 490 B1). Peptide nucleic acids (PNA) represents a class of nucleic acid analogues where the charged deoxyribose-phosphodiester backbone has been replaced by a peptide strand comprising N-(2-aminoethyl)glycine monomers with the nucleobases adenine, thymine, guanine and cytosine attached to their alpha-carbon. Due to its artificial character PNA provides complete resistance against nucleases and proteases.

In a preferred embodiment, combinations of two or more aptamers with specificity for IL-4 and IL-13 which are capable of blocking the biologic activity of both cytokines, are used for the method of the present invention.

In another preferred embodiment of the invention, aptamers capable of inactivating cytokine receptors are used to inhibit IL-4- and IL-13-mediated pathways. Preferred are aptamers with specificity for IL-4Rα which are capable of blocking the biologic activity of both IL-4 and IL-13.

In another preferred embodiment of the invention, aptamers are used which provide limited resistance against enzymatic degradation that is sufficient for effective inhibition of IL-4- and IL-13-mediated pathways at the site of allergen presentation and in its close proximity, but guarantees rapid degradation of the aptamer while diffusing away from this site. In a specific embodiment, the stability of an inhibitory aptamer is adjusted according to the requirement of the method of the present invention by modification of its structure including but not limited to the introduction of phosphorothioate linkages, modification of bases (e.g., by modification with 2'-aminopyrimidine), and addition of a 3'-3' linkage to the end of the aptamer.

In another preferred embodiment of the invention, the physiological clearance of an inhibitory aptamer is adjusted according to the requirement of the method of the present invention. In cases of an extremely fast clearance, the plasma life-time of an inhibitory aptamer can be extended by attachment of lipids or high molecular weight compounds such as 40 kD polyethylene glycol moieties.

### VI. Matrices for prolonged delivery of inhibitors of decision signals for T-cell differentiation

In a preferred embodiment, matrices are used for local delivery of inhibitors of IL-4- and IL-13-mediated pathways that a) can serve as depot for substantial quantities of the inhibitor or inhibitor combinations, b) allow the release of sufficient quantities of the inhibitor or inhibitor combinations for efficient local inhibition of IL-4- and IL-13-mediated pathways over a prolonged period of time (optimally for a few days), c) are biodegradable, and d) are chemically and physically compatible with the adjuvant and allergen (s) used for the induction of tolerance according to the method of the present invention.

In one embodiment of the invention, biodegradable polymers are used for controlled delivery of inhibitors of IL-4- and IL-13-mediated pathways. Preferred biodegradable polymers approved by FDA and used in a clinical trial, include but are not limited to poly(D,L-lactic acid), poly(lactic-coglycolic acid) (PLGA), and copolymers of L-lactide and D,L-lactide. An important characateristic of such polymers is their ability to be applied locally which allows intralesional concentrations of the inhibitors to be sustained while systemic deleterious side effects are minimized. All FDA approved polymers have been studied extensively for their biocompatibility, toxicology, and degradation kinetics. Furthermore, these polymers have been shown to release embedded therapeutics for several hours up to 40 weeks in vitro and are effective for several weeks *in vivo.* For example, PLGA microspheres containing the anti-VEGF RNA aptamer EYE001 have been demonstrated to deliver the aptamer in a sustained manner with an average rate of 2 µg per day over a period of 20 days (Carrasquillo *et al*., 2003).

In a more preferred embodiment, injectable in situ-forming gel systems which are biodegradable, are used for controlled delivery of inhibitors of IL-4- and IL-13-mediated pathways. Preferred in situ-forming gel systems (hydrogels) undergo a sol-gel-sol transition, which is free flowing sol at room temperature and a non-flowing gel at body temperature. Compared to other biodegradable polymers, the injectable thermogelling polymers possess several advantages including easy preparation, high encapsulation efficiency of bioactive molecules such as inhibitors of IL-4- and IL-13-mediated pathways, and free of harmful organic solvents in the formulation process (Qiao *et al*. 2005).

In one specific embodiment, biodegradable thermogelling block polymers are used which are based on monomethoxy poly(ethylene glycol) (MPEG) including but not limited to a) diblock copolymers consisting of MPEG and poly(ε-caprolactone) (PCL) (Hyun *et al*., 2007), b) MPEG-*b*-(PCL-*ran*-PLLA) diblock copolymers (Kang *et al*., 2010), and c) diblock copolymers consisting of MPEG and PLGA (Peng *et al*., 2010). MPEG copolymers containing PCL provide the advantage that they do not create an acidic environment upon biodegradation in contrast to MPEG copolymers containing PLLA and PLGA (Hyun *et al*., 2007).

In another specific embodiment, biodegradable thermogelling triblock polymers are used including but not limited to a) PLGA-PEG-PLGA (Qiao *et al.,* 2005), b) PEG-PLGA-PEG (Zhang *et al*., 2006), and c) PEG-PCL-PEG (PECE) (Gong *et al*., 2009 a). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

In another specific embodiment, biodegradable thermogelling diblock and triblock copolymers are used which consist of polyethylene oxide (PEO) and a biodegradable polyester such as poly-L-lactic acid (PLLA) (Jeong *et al*., 1997). These block copolymers, however, are a free flowing sol at a higher temperature and form a gel at a lower temperature. For example, a 23% aqueous solution of PEO-PLLA-PEO (Mᵣ 5,000-2,040-5,000) is a sol at 45°C and becomes a gel at 37°C By changing the biodegradable block length, the sol-gel transition temperature can be manipulated, e.g., increasing the PLLA block length increases the aggregation tendency of a block copolymer in water, resulting in a steepening of the gel-sol transition curve slopes and the onset of gelation at lower concentrations. The sol-gel transition temperature is a function of concentration as well as composition of a block polymer.

In still another specific embodiment, Poloxamers (trade name Pluronics)are used. Poloxamers are nonionic triblock copolymers composed of a central hydrophobic chain of poly(propylene oxide) (PPO) flanked by two hydrophilic chains of poly (ethylene oxide) (PEO) (Gilbert *et al*., 1987). Poloxamers exhibit also sol-gel transition behavior in aqueous solutions and have been used for sustained delivery of several therapeutic agents. However, Poloxamers are not biodegradable and can be accumulated in the body which may lead to toxic side effects. Thus, the application of Poloxamers in biomedical fields has been greatly restricted. In a recent study, Pluronic F127 (100-unit PEO chain surrounding one 65-unit PPO) has been used to form composite thermosensitive hydrogels with PECE (Gong *et al*., 2009-b). Based on the results of this study Pluronic F127/PECE composite hydrogels are biocompatible with low cell cytotoxicity and, therefore, may also be suitable for the method of the present invention.

The various biodegradable thermogelling polymers provide different stability characteristics. For example, Poloxamer triblock polymers provide excellent thermosensitivity, but due to weak hydrophobicity of the PPO block such copolymers form fast eroding gels which have been reported to persist in vivo a few hours at most. Exposure of Poloxamer gels to phosphatebuffered saline under in vitro conditions demonstrated gel erosion within 2 days (Hyun *et al*., 2007). Similar results were observed when the polymer solutions were subcutaneously injected into rats. Poloxamer gels could not be observed after 2 days (Hyun *et al*., 2007). Different results were obtained with MPEG-PCL gels. Under in vitro conditions MPEG-PCL gels maintained their structural integrity for more than 28 days and after subcutaneous injection into rats MPEG-PCL gels maintained their structural integrity longer than 30 days. The stability of MPEG-PCL gels, however, may also create problems since the rate of degradation of PCL *in vivo* is rather slow (2-3 years) compared to that of PLA, PGA or PLGA (for a review, see Sinha *et al*., 2004). Thus, after serving the function in delivering inhibitors of IL-4- and IL-13-mediated pathways *in vivo*, MPEG-PCL copolymers may remain in the body under physiological conditions for an uncertain period. Therefore, most preferred biodegradable thermogelling polymers for the method of the present invention are those which maintain their structural integrity for a few days but do not remain in the body for more than a week.

In a preferred embodiment of the present invention, biodegradable thermogelling polymers are used which allow to modify their degradation kinetics. For example, PLLA segments can be incorporated into the PCL segment of MPEG-PCL copolymers, since PLLA provides better accessibility of water to the ester bonds of PLLA which enhances the hydrolytic degradation of the copolymer (Kang *et al*., 2010). The resulting MPEG-*b*-(PCL-*ran*-PLLA) diblock copolymers offer a therapeutic window that is adjustable from a few weeks to a few months by varying the amount of PLLA in the PCL segment (Kang *et al*., 2010). In another example, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblock copolymers, more stable hydrogels are formed due to stronger hydrophobic interactions among the copolymer molecules (Qiao *et al*. 2005).

Several of the biodegradable thermogelling polymers have been analyzed for their ability to mediate sustained release of proteins. Although different proteins such as cytokine mutants, soluble cytokine receptor constructs and antibodies are likely to affect the release behavior of each copolymer in individual ways, characterization of the release of model proteins such as bovine serum albumin (BSA) provides important information. Using composite hydrogels containing different percentages of PECE and Pluronic F127, the in vitro release behavior of BSA proved to be dependent on the hydrogel composition, initial BSA loading amount, and hydrogel concentration (Gong *et al.,* 2009-b). Sustained release of BSA above 15 days was achieved with a composite hydrogel containing 60% PECE and 40% Pluronic F127, loaded with 4 mg BSA in the presence of 30wt% hydrogel. Using MPEG-PCL copolymers, sustained *in vitro* release of BSA proved to be above 20 days, and under *in vivo* conditions (after subcutaneous injection into rats) sustained release lasted for more than 30 days (Hyung *et al.,* 2007).

While for most clinical applications a sustained release of therapeutic drugs from biodegradable thermogelling polymers over a period of several weeks is desirable, the method of the present invention does not require such an extended sustained release of inhibitors of IL-4- and IL-13-mediated pathways since the development of immunologic memory requires the engagement of the T cell receptor (TCR) for a period of only 1 to 2 days. Furthermore, an extended sustained release of inhibitors of IL-4- and IL-13-mediated pathways over a period of several weeks may create local adverse effects. Therefore, preferred are biodegradable thermogelling polymers which deliver inhibitors of IL-4- and IL-13-mediated pathways for a limited period only which does not exceed a week.

PLGA-PEG-PLGA triblock copolymers represent one example of hydrogels providing advantageous degradation and release kinetics for the method of the present invention. As demonstrated in a recent study, the release of insulin from PLGA-PEG-PLGA triblock copolymers lasted for approximately 4 days with an initial burst effect during the first day (Choi *et al,* 2003). The initial burst effect may be advantageous for complete inhibition of IL-4- and IL-13-mediated pathways in the initial phase of allergen presentation. However, the release kinetics may be modified by reducing the solubility of inhibitors of IL-4- and IL-13-mediated pathways via complexation with zink as shown for zink-insulin complexes (Choi *et al,* 2003).

Although Poloxamer gels are not biodegradable, Poloxamer 407 (trade name Pluronic F127) gels represent also an example of thermogelling polymers providing advantageous degradation and release kinetics for the method of the present invention. Although under *in vitro* conditions the release of BSA from Poloxamer 407 gels proved to be almost complete within 1 day, the sustained release of BSA under *in vivo* conditions (after subcutaneous injection into rats) lasted for 3 days (Hyung *et al., 2007).*

### VII. Adjuvants

The time of antigen exposure is extremely important since T cell differentiation and thus the development of immunologic memory requires the engagement of the T cell receptor (TCR) over 12-48 h and long lasting memory may even require repetitive exposure. Adjuvants such as oils and alum provide a depot effect that extends the lifetime of antigens and thus prolong antigen-stimulation of T cells. However, in addition to the depot effect currently available adjuvants trigger either Th1-type or Th2-type immune responses or both (for reviews, see Leroux-Roels, 2010; Nicholls *et al.,* 2010). Based on the assumption that the generation of Tregs represents a default pathway which requires T-cell receptor activation but the absence of decision signals for effector T cells, currently available adjuvants are not optimal for the purpose of the present invention. For the method of the present invention adjuvants are needed that promote allergen uptake and prolonged stimulation of T cells without providing decision signals, thereby inducing Treg expansion. Unfortunately, adjuvants providing all of the desired properties are not available.

Liposomes, synthetic nanospheres consisting of lipid layers, represent a classic adjuvant that is not active per se. Their main mode of action is through allergen presentation. Liposomes, can encapsulate allergens and act as allergen delivery vehicles. However, liposomes are not suitable for a slow release of allergens, which appears to be essential for a prolonged immune stimulation.

In a preferred embodiment of this invention, adjuvants eliciting Th2-type immune responses including but not limited to aluminum salts, Montanide emulsions (squalene-based water-in-oil emulsions) and polyphosphazenes, are used for the method of the present invention. Although adjuvants elicting primarily a Th2-type immune response are potentially interfering with the induction of Tregs, in the presence of inhibitors of Il-4- and IL-13-mediated pathways such adjuvants are better suited for the therapeutic aims of the present invention than those eliciting primarily a Th1-type immune response. In the presence of inhibitors of Il-4- and IL-13-mediated pathways the contra-productive activity of adjuvants eliciting Th2-type immune responses is significantly reduced or even abolished, whereas the activity of Th1-type promoting adjuvants cannot be influenced. As a result, Th1-type cytokines would interfere with the induction of Tregs.

In one preferred specific embodiment, aluminum-containing adjuvants, typically aluminum phosphate or aluminum hydroxide gels (generally referred to as alum) are used for the method of the present invention. One mechanism of action is due to its retention of immunogenic molecules at specific sites in the body, allowing for their slow release and consequently a prolonged immune response, the so-called depot effect. Alum also appears to exert its adjuvantic activity by keeping antigens in a particulate (rather than soluble) form, thereby enhancing phagocytosis of antigens by APCs. Dendritic cells (DC) are capable of taking up both soluble antigens and antigens adsorbed onto aluminum-containing adjuvants. Furthermore, it has become evident that alum is capable of directly activating immune cells. Alum can induce maturation of monocytes/macrophages into DC-like cells. This process is dependent on the activation of NLRP3 (NOD-like receptor family, pyrin domain containing 3), part of the inflammasome which causes production of pro-inflammatory cytokines such as IL-1β and IL-18. Primarily, alum elicits a Th2 response including IL-4 and IL-5 production as well as IgG1 and IgE production by B cells (for a review, see Nicolls *et al*., 2010).

Upon incubation of allergens with aluminum-containing adjuvants, the allergens are partially or completely adsorbed onto the adjuvant via electrostatic interactions and exchange between phosphate and hydroxyl groups. Several studies have demonstrated that adsorption onto aluminum-containing adjuvants prevents rapid enzymatic degradation of the allergens, but the adsorption process may also induce structural changes resulting in a decreased thermal stability (for a review, see Clapp *et al*., 2011). One approach to improving the thermal stability of vaccines is to use a combination of buffers to alter the surface chemistry of aluminum-containing adjuvants and to control the ionization state of the allergen's amino acid side chains. Another approach is to identify conditions that stabilizes the allergen(s) in the solution state and to use this as guidance on minimizing the extent of adsorption-induced thermal destabilization of the allergen(s). For practical purposes, however, stabilization procedures may be omitted since the importance of antigen (including allergen) adsorption to aluminum-containing adjuvants for the immunological response has been questioned by recent studies (for a review, see Clapp *et al*., 2011). For example, the immune response elicited in rabbits against hen egg white lysozyme was irrespective of the fraction of antigen adsorbed onto the aluminum-containing adjuvant (Chang *et al*., 2001). For maximizing the immune response, however, some degree of adsorption or at least the proximity of the allergen and the adjuvant appears to be important.

In another preferred specific embodiment, polyphosphazenes (water-soluble polymers) are used for the method of the present invention. Poly [di(carboxylatophenoxy) phosphazene] (PCPP) has been shown to enhance antibody responses while being negligibly reactogenic (Nicolls *et al.,* 2010).

In still another specific embodiment, squalene-based water-in-oil emulsions (Montanide emulsions) are used for the method of the present invention. Montanide emulsions are similar to incomplete Freund's adjuvant, but they are biodegradable and therefore significantly less toxic and have been used in several clinical trials (Nicolls *et al*., 2010).

In another embodiment, adjuvants eliciting a combined Th1- and Th2-type immune resonse are used for the method of the present invention. Although adjuvants eliciting a combined Th1- and Th2-type immune resonse are likely to interfere to a certain extent with the induction of Tregs, such adjuvants may provide advantageous properties for the composition, which may be beneficial for the method of the present invention. Adjuvants eliciting a combined Th1-typ and Th2-type immune response include but are not limited to squalene-based oil-in-water emulsions, granulocyte-macrophage colony stimulating factor (GM-CSF), and adjuvants based on inulin and virosomes (for a review, see Nicholls *et al.,* 2010).

### VIII. Vaccine compositions comprising inhibitors of IL-4- and IL-13-mediated pathways, a matrix mediating prolonged delivery of such inhibitors, allergens and adjuvants.

In order to achieve prolonged presentation of the allergen(s) along with a prolonged inhibition of IL-4- and IL-13-mediated effects at the site of allergen presentation, both the allergen(s) and inhibitor(s) are co-administered in a manner that limits diffusion of these components away from the site of allergen presentation. Useful diffusion limiting techniques for the method of the present invention include but are not limited to adsorption of components onto aluminum-containing materials, preparation of water-in-oil or oil-in-water emulsion with components, and incorporation of components into biodegradable polymers including biodegradable thermogelling polymers.

For some components it is useful to combine two or more of the diffusion limiting techniques to achieve the therapeutic aims of the present invention. One important aspect of the method of the present invention is the compatibility of the components in the therapeutic composition For example, adsorption of allergens and inhibitors of IL-4- and IL-13-mediated pathways onto aluminum-containing materials provides a prolonged presentation of the allergens together with a Th2-type adjuvant effect, but it is questionable whether the delayed release of the inhibitor(s) is sufficient for effective inhibition of IL-4- and IL-13-mediated pathways during allergen presentation. Incorporation of the inhibitors into a fast degrading polymer provides a better opportunity to achieve effective inhibition of IL-4- and IL-13-mediated pathways during allergen presentation. Therefore, a combination of both techniques is likely to guarantee a higher therapeutic efficacy than each of the techniques alone. Similar considerations apply to the use of water-in-oil or oil-in-water emulsion as diffusion limiting technique for inhibitors of IL-4- and IL-13-mediated pathways, since the release kinetics of inhibitors from such emulsions may not be sufficient for the requirements of the method of the present invention. However, biodegradable polymers have the potential to provide both prolonged allergen presentation and a sustained release of inhibitors of IL-4- and IL-13-mediated pathways sufficient for the requirements of the method of the present invention. A recent study has demonstrated that soluble adjuvants embedded together with antigens in biodegradable hydrogels are potent compositions for eliciting immune responses. Using diblock copolymers consisting of MPEG-PLGA for hydrogel-codelivery of hepatitis B surface antigen (HBsAg) and GM-SCF, it was possible to elicit high HBsAg-specific antibodies and T-helper cell responses even in a mouse strain that does not respond to current HBsAg vaccine because of its H-2 haplotype (Chou *et al*., 2010).

In one preferred embodiment of the present invention, the vaccine composition comprises a biodegradable thermogelling polymer containing one or more allergens or an allergen extract partially or completely adsorbed onto aluminum-containing adjuvants, and one or more soluble inhibitors of IL-4- and IL-13-mediated pathways. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In another preferred embodiment of the present invention, polymer-embedded inhibitors of IL-4- and IL-13-mediated pathways and alum-adsorbed allergens (or allergen extract) are injected at the same location, but as separate preparations. After gellation of the polymer composit at the injection site, alum-adsorbed allergens (or allergen extract) are injected in close proximity of the gelled polymer composit. The polymer composit comprises a biodegradable thermogelling polymer and one or more soluble inhibitors of IL-4- and IL-13-mediated pathways, and is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In another preferred embodiment of the present invention, the vaccine composition comprises a biodegradable thermogelling polymer containing one or more allergens or an allergen extract premixed with an adjuvant consisting of a squalene-based water-in-oil emulsion (Montanide emulsion), or a water-soluble polyphosphazenes polymer, and one or more soluble inhibitors of IL-4- and IL-13-mediated pathways. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In another preferred embodiment of the present invention, polymer-embedded inhibitors of IL-4- and IL-13-mediated pathways, and allergens (or allergen extract) either embedded in a Montanide emulsion or mixed with a water-soluble polyphosphazenes polymer are injected at the same location, but as separate preparations. After gellation of the polymer composit at the injection site, the allergens (or allergen extract) either embedded in a Montanide emulsion or mixed with a water-soluble polyphosphazenes polymer are injected in close proximity of the gelled polymer composit. The polymer composit comprises a biodegradable thermogelling polymer and one or more soluble inhibitors of IL-4- and IL-13-mediated pathways, and is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In still another preferred embodiment of the present invention, the vaccine composition comprises a biodegradable thermogelling polymer containing one or more allergens or an allergen extract, one or more soluble adjuvants eliciting a combined Th1- and Th2-type immune resonse (such as GM-CSF), and one or more soluble inhibitors of IL-4- and IL-13-mediated pathways. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

### IX. Therapeutic methods

As used herein, a decrease or modification of the T cell response of an individual sensitive to a protein allergen is defined as diminution in symptoms to the protein allergen in the individual, as determined by standard clinical procedures (see e.g., Brit. Med. J. 1990; 302: 265). As referred to herein, a diminution in symptoms to an allergen includes any reduction in the allergic response of an individual to the allergen following a treatment regimen with a polypeptide as described herein. This diminution in symptoms may be determined subjectively (e.g., the patient feels more comfortable upon exposure to the allergen), or clinically, such as with a standard skin test.

For immunotherapy, the vaccine compositions of the present invention comprising one or more allergens, one or more adjuvants, one or more inhibitors of IL-4- and IL-13-mediated pathways, and a matrix mediating prolonged delivery of such inhibitors, are repeatedly administered at intervals as defined in various published immunotherapy protocols. The dose of the allergen or allergens in the vaccine compositions is gradually increased to an extent that is effective to reduce the allergic response of the individual to the allergen(s). In a particular embodiment, non-embedded inhibitors of IL-4- and IL-13-mediated pathways may be co-administered with the vaccine compositions to enhance the inhibitory effect at the site of administration. Preferred examples of routes of administration include but are not limited to intradermal and subcutaneous administrations. Dosage regimens may be adjusted to provide the optimum therapeutic response. For example, several divided doses may be administered daily.

In one preferred embodiment of the present invention, polymer-embedded inhibitors of IL-4- and IL-13-mediated pathways and alum-adsorbed allergens are applied for immunotherapy. First, a biodegradable thermogelling polymer solution containing one or more soluble inhibitors of IL-4-and IL-13-mediated pathways is injected subcutaneously. The quantity of the polymer-embedded inhibitors will depend on the release kinetics of the biodegradable thermogelling polymer and is adjusted to a level that guarantees the continuous release of therapeutically effective doses over a period of 3 to 5 days. In a second step, alum-adsorbed allergens are injected subcutaneously at the site of the gelled polymer composit. The quantity of alum-adsorbed allergens will vary according to factors such as the degree of sensitivity of the individual to the allergen(s), the age, sex, and weight of the individual, and the ability of the composition to induce tolerance toward the allergen(s) in the individual. In subsequent steps, polymer-embedded inhibitors are injected first, followed by the injection of alum-asorbed allergens at the site of the gelled polymer composit. While the quantity of polymer-embedded inhibitors remains constant, the quantity of alum-adsorbed allergens is gradually increased to an extent that is effective to reduce the allergic response of the individual to the allergens. Both polymer-embedded inhibitors and alum-adsorbed allergens are repeatedly administered at intervals as defined in various published immunotherapy protocols until allergen tolerance is achieved.

The determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

### X. Pharmaceutical formulations

In one embodiment, the vaccine compositions are incorporated into pharmaceutical compositions suitable for administration. Such compositions typically comprise the vaccine composition and a pharmaceutically acceptable carrier. As used herein, a 'pharmaceutically acceptable carrier' is intended to include any and all solvents, dispersion media, coatings, antibacterial and antifungal agents, isotonic systems, and the like, compatible with the components of the vaccine composition and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition. The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. For practical purposes it should be kept in mind that aluminum-adsorbed vaccines are frost sensitive and therefore not lyophilizable.

### References cited and incorporated by reference herein

Andersson, A., et al. Mouse macrophage development in the absence of the common γ chain: defining receptor complexes responsible for IL-4 and IL-13 signaling. Eur. J. Immunol. 27: 1762-1768; 1997.
Andrews, A.-L., et al. IL-4 receptor alpha is an important modulator of IL-4 and IL-13 receptor binding: implications for the development of therapeutic drugs. J. Immunol. 176: 7456-7461; 2006.
Borish, L.C., et al. Efficacy of soluble IL-4 receptor for the treatment of adults with asthma. J. Allergy Clin. Immunol. 107: 963-970; 2001.
Carrasquillo, K.G., et al. Controlled delivery of the anti-VEGF aptamer EYE001 with poly(lactic-co-glycolic) acid microspheres. Invest. Ophthalmol. Vis. Sci. 44: 290-299; 2003.
Chang, M., et al. Degree of antigen adsorption in the vaccine or institial fluid and its effect on the antibody response in rabbits. Vaccine 19: 2884-2889; 2001.
Choi, S., et al. Controlled release of insilin from injectable biodegradable triblock copolymer depot in ZDF rats. Pharmaceut. Res. 20: 2008-2010; 2003.
Chou, H.-Y., et al. Hydrogel-delivered GM-CSF overcomes nonresponsiveness to hepatitis B vaccine through the recruitment and activation of dendritic cells. J. Immunol. 185(9): 5468-5475; 2010.
Clapp, T., et al. Vaccines with aluminum-containing adjuvants : optimizing vaccine efficacy and thermal stability. J. Pharm. Sci. 100: 388-401; 2011.
Corren, J., et al. A randomized, controlled, Phase 2 study of AMG 317, an IL-4Rα antagonist, in patients with asthma. Am. J. Respir. Crit. Care Med. 181: 788-796; 2010.
Duschl, A., et al. A comparison of assays for the response of primary human T-cells upon stimulation with interleukin-2, interleukin-4 and interleukin-7.Eur. Cytokine Netw. 3: 97-102; 1992.
Economides, A.E., et al. Cytokine traps: multi-component, high affinity blockers of cytokine action. Nature Med. 9: 47-52; 2003.
Famulok, M., et al. Aptamers as tools in molecular biology and immunology. Curr. Top. Microbiol. Immunol. 243: 123-136; 1999.
Gauvreau, G.M., et al. The effects of IL-13 blockade on allergen-induced airway responses in mild atopic asthma. Am. J. Respir. Crit. Care Med.; accepted Nov 5 2010, in print.
Gessner, A., et al. Biologic functions and signaling of the interleukin-4 receptor complexes. Immunobiology 201(3-4): 285-307; 2000.
Gilbert, J.C., et al. The effect of solutes and polymers on the gelation properties of Pluronic F-127 solutions for controlled drug delivery. J. Control. Release 5: 113-118; 1987.
Gong, C.Y., et al. Synthesis, characterization, degradation, and in vitro drug release behavior of thermosensitive hydrogel based on PEG-PCL-PEG block copolymers. Int. J. Pharm. 365: 89-99; 2009 a.
Gong, C.Y., et al. In vitro drug release behavior from a novel thermosensitive composite hydrogel based on Pluronic f127 and poly(ethylene glycol)-poly (ε-caprolactone)-poly(ethylene glycol) copolymer. BMC Biotechnol. 9: 8; 2009 b.
Grunewald, S.M., et al. A murine interleukin-4 antagonistic mutant protein completely inhibits interleukin-4-induced cell proliferation, differentiation, and signal transduction. J. Biol. Chem. 272: 1480-1483; 1997.
Grunewald, S.M., et al. An antagonistic IL-4 mutant prevents type I allergy in the mouse: inhibition of the IL-4/IL-13 receptor system completely abrogates humoral immune response to allergen and development of allergic symtoms in vivo. J. Immunl. 160: 404-4009; 1998.
Grote, A., et al. JCat: a novel tool to adapt codon usage of a target gene to its potential expression host. NAR 33: W526-531, 2005.
Hart, T.K., et al. Preclinical efficacy and safety of pascolizumab (SB 240683): a humanized anti-interleukin-4 Antibody with therapeutic potential in asthma. Clin. Exp. Immunol. 130: 93-100; 2002.
Holgate, S.T., et al. Treatment strategies for allergy and asthma. Nat. Rev. Immunol. 8: 218-230; 2008.
Hyun, H., et al. In vitro and in vivo release of albumin using a biodegradable MPEG-PCL diblock copolymer as an in situ gel-forming carrier. Biomacromolecules 8: 1093-1100; 2007.
Idzerda et al. Human interleukin 4 receptor confers biological responsiveness and defines a novel receptor superfamily. J. Exp. Med. 171(3): 861-873; 1990.
Jeong, B., et al. Biodegradable block copolymers as injectable drug-delivery systems. Nature 388: 860-862, 1997.
Kang, Y.M., et al. A biodegradable, injectable gel system based on MPEG-b-(PCL-ran-PLLA) diblock copolymers with an adjustable therapeutic window. Biomaterials 31: 2453-2460; 2010.
Karamloo, F., et al. Prevention of allergy by a recombinant multi-allergen vaccine with reduced IgE binding and preserved T cell epitopes. Eur. J. Immunol. 35:3268-3276; 2005.
Kioi, M., et al. Mechanism of action of interleukin-13 antagonist (IL-13E13K) in cells expressing various types of IL-4R. Cell. Immunol. 229: 41-51; 2004.
Kruse, N., et al. Conversion of human interleukin-4 into a high affinity antagonist by a single amino acid replacement, EMBO J. 11(9): 3237-3244; 1992.
Kruse, N., et al. Two distinct functional sites of human interleukin 4 are identified by variants impaired in either receptor binding or receptor activation. EMBO J. 12: 5121-5129; 1993.
Leroux-Roels, G. Unmet needs in modern vaccinology Adjuvants to improve the immune response. Vaccine 285: C25-C36; 2010.
Letzelter, F., et al. The interleukin-4 site-2 epitope determining binding of the common receptor γ-chain. Eur. J. Biochem. 257: 11-20; 1998.
Long, A.A. Monoclonal antibodies and other biologic agents in the treatment of asthma. MAbs 1(3), 237-246; 2009.
Mantel, P.-Y., et al. Molecular mechanisms underlying FOXP3 induction in human T cells. J. Immunol. 176: 3593-3602; 2006.
Mantel, P.-Y. et al. GATA3-driven Th2 responses inhibit TGF-β1-induced FOXP3 expression and the formation of regulatory T cells. PLoS biology 5, e329: 2847-2861; 2007.
Muller, T., et al. Aspects of receptor binding and signalling of interleukin-4 investigated by site-directed mutagenesis and NMR spectroscopy. J. Mol. Biol. 237 : 423-436, 1994.
Mosley, B., et al., The murine interleukin-4 receptor : Molecular cloning and characterization of secreted and membrane bound forms. Cell 59: 335-348; 1989.
Nandakumar, S., et al. T regulatory cells: an overview and intervention techniques to modulate allergy outcome. Clin. Mol. Allergy 7: 1-8; 2009.
Nicholls, E.F., et al. Immunomodulators as adjuvants for vaccines and antimicrobial therapy. Ann. N.Y. Acad. Sci. 1213: 46-61; 2010.
Niederberger, V., et al. Vaccination with genetically engineered allergens prevents progression of allergic disease. Proc. Natl. Acad. Sci. USA 101:14677-14682; 2004.
Noma Y., et al. Cloning of cDNA encoding the murine IgG1 induction factor by a novel strategy using SP6 promotor. Nature 319: 640-646, 1986.
Peng, K.-T., et al. Treatment of osteomyelitis with teicoplanin-encapsulated biodegradable thermosensitive hydrogel matrices. Biomaterials 31: 5227-5236; 2010.
Puigbo P., et al. OPTIMIZER: a web server for optimizing the codon usage of DNA sequences. NAR 35: W126-131, 2007.
Qiao, M. et al. Injectable biodegradable temperatureresponsive PLGA-PEG-PLGA copolymers: synthesis and effect of copolymer composition on the drug release from the copolymer-based hydrogels. Int. J. Pharm. 294: 103-112; 2005.
Razeghifard, M.R. On-column refolding of recombinant human interleukin-4 from inclusion bodies. Prot. Expr. Purif. 37: 180-186; 2004.
Schmidt-Weber, C., Blaser, K. New insights into the mechanisms of allergen-specific immunotherapy. Curr. Opin. Allergy Clin. Immunol. 5: 525-530; 2005.
Schmidt-Weber C., Blaser, K. Immunological mechanisms of specific allergen immunotherapy. Inflamm. Allergy Drug Targets 5: 15-21; 2006.
Schubert, L.A., et al. Scurfin (FOXP3) acts as a repressor of transcription and regulates T cell activation. J. Biol. Chem. 276: 37672-37679; 2001.
Sinha, V.R., et al. Poly-epsilon-caprolactone microspheres and nanospheres : an overview. Int. J. Pharm. 278: 1-23; 2004.
Tony, H.P., et al. Design of human interleukin-4 antagonists inhibiting interleukin-4-dependent and interleukin-13-dependent responses in T-cells and B-cells with high efficiency. Eur. J. Biochem. 225: 659-665; 1994.
Wenzel, S., et al. Effect of interleukin-4 variant on late phase asthmatic response to allergen challenge in asthmatic patients: results of two phase 2a studies. Lancet 370: 1422-1431; 2007.
Wong, H.L., et al. Administration of recombinant IL-4 to humans regulates expression, phenotype, and function in circulating monocytes. J. Immunol. 148: 2118-2125,; 1992.
Yan, A.C., et al. Aptamers: prospects in therapeutics and biomedicine. Frontiers Biosci. 10: 1802-1827; 2005.
Zhang, J., et al. Micellization phenomena of amphiphilic block copolymers based on methoxy poly(ethylene glycol) and either crystalline or amorphous poly(caprolactone-b-lactide). Biomacromolecules 7: 2492-2500; 2006.
EP 1 785 490 B1 (Method for determining an unknown PNA sequence and uses thereof)
WO 97/47744 (T-cell selective interleukin-4 agonists)
WO 99/18142 (Biodegradable low molecular weight triblock poly(lactide-co-glcolide) polyethylene glycol copolymers having reverse thermal gelation properties)
WO 02/04009 (Method for treating cancer)
WO 2009/081 201 (Binding members for interleukin-4 receptor alpha-173)
US 2011/0002913 (Use of interleukin-4 antagonists and compositions thereof)
US 6,028,176 (High-affinity interleukin-4 muteins)
US 5,723,118 (Therapeutic agents which are antagonists or partial agonists of human interleukin 4)
US 5,599,905 (Interleukin-4 receptors)

### EXAMPLES

### EXAMPLE 1: Cloning, expression and purification of the murine IL-4 antagonist QY

A major obstacle for testing the effects of human antagonistic IL-4 mutants in vivo is the species specificity of IL-4. Human IL-4 does not bind to the mouse receptor and vice versa. Therefore, the murine IL-4 antagonist QY (Q116D/Y119D) in which the amino acids glutamine 116 and tyrosine 119 are mutated to aspartic acid, has been generated for the proof of concept. This murine IL-4 mutant is analogous to the R121D/Y124D double mutant of human I1-4, binds with high affinity to the murine IL-4Ra without inducing signal transduction, has no detectable activity upon proliferation or differentiation of murine cells, and an excess of the murine QY mutant has been shown to completely inhibit responses toward wild-type murine IL-4 (Grunewald *et al*., 1997). Furthermore, the murine QY mutant is also a complete inhibitor for murine IL-4 in the absence of γ (Andersson *et al*., 1997).

### Cloning of the murine IL-4 antagonist QY (Q116D/Y119D).

The sequence of the native mature murine IL4 protein is modified to include a thrombin cleavable N-terminal 6xHis-tag and the QY (Q116D/Y119D) mutations (see SEQID 10). The protein sequence is translated into DNA using codon optimization for expression in prokaryontic *E*. *coli* cells (Puigbo *et al*, 2007; Grote *et al*, 2005). The resulting sequence (SEQID 11) is synthesized and amplified with primers 1 (IL4his-for) and 3 (MOIL4mut-back) using PCR with Pfu polymerase according to the manufacture and annealing temperatures rising from initial 5 cycles at 50°C to 60°C and 30 cycles total. The resulting PCF fragment is gel-purified in 1% agarose (GeneJET Gel Extraction Kit, Fermentas) and transferred to a restriction enzyme double digest containing Nde *I* and *Xho I* (Fermentas). The restriction digest is again gel-purified and the fragment is ligated into *Nde I* and *Xho I* cut pET-22b (Novagen) expression vector.

### Expression.

Recombinant His-tagged murine IL-4 antagonist QY (Q116D/Y119D) is expressed in *E. coli* (BL21(DE3)pLysS; Novagen) at 37°C. Cultures are grown in 1-L batches in minimal medium containing 100 µg/ml ampicillin and 34 µg/ml chloramphenicol. The minimal medium contains the following components: 0.1 M phosphate buffer, pH 7.0, 2 mM MgSO₄, 17 mM NaCl, 0.1 mM CaCl₂, 5 mg/L FeSO₄, 0.2 mg/L (NH₄)MO₇O₂₄, 1 mg/L H₃BO₃, 0.2 mg/L CoCl₂, 0.2 mg/L CuSO₄, 2 mg/L MnSO₄, and 2 mg/L ZnSO₄, supplemented with thymidine, biotin, folic acid, pantothenic acid, niacinamide, pyroxidine phosphate, and thiamine (1 mg/L each). The expression is induced by the addition of isopropyl-D-thiogalactopyranoside to a final concentration of 1 mM after the culture reaches an OD₆₀₀ of approximately 0.6. The cells are allowed to grow for 4h and then harvested by centrifugation at 5000 x g for 10 min at 4°C. The pellet is stored at -20°C.

### Refolding from inclusion bodies.

The insoluble His-tagged murine IL-4 antagonist QY (Q116D/Y119D) is refolded according to a published protocol (Razeghifard, 2004). The *E*. *coli* pellet containing the recombinant IL-4 antagonist QY is resuspended in 15 ml of 50 mM Tris-HCl, pH 8.0, and 5 mM EDTA (lysis buffer) containing 2 mg lysozyme and incubated at room temperature for 20 min. The cell lysate is diluted with 60 ml lysis buffer and sonicated. It is then centrifuged at 11,000 rpm for 30 min at 4°C to collect the pellet containing inclusion bodies. The pellet is washed twice with 60 ml with 2 M urea in lysis buffer and collected by centrifugation as described above. Finally, the pellet is resuspended in 25 ml of 6 M GdnHCl, 50 mM potassium phosphate, pH 8.0, and 1 mM reduced glutathione (extraction buffer) and stirred at room temperature for 10 min. The solution is centrifuged to remove the insoluble portion.

The guanidine-extracted IL-4 antagonist QY is loaded onto 15 ml of Ni-charged HisTrap™ FF columns (GE Healthcare) equilibrated with the extraction buffer. The beads are washed with 8 column volumes of 7 M urea and 50 mM potassium phosphate, pH 6.8, to remove the cellular proteins. The His-tagged IL-4 antagonist QY is refolded on the column by the gradual removal of urea through a linear gradient expanding from 100% unfolding buffer to 100% refolding buffer (380 ml at a flow rate of 1 ml/min) using a fast protein liquid chromatography ÄKTA™ system (Pharmacia). The unfolding buffer is composed of 7 M urea, 100 mM NaCl, 50 mM potassium phosphate, pH 6.8, 1 mM reduced glutathione, and 1 mM oxidized glutathione; the refolding buffer has the same composition but without urea. After washing the column with 40 ml refolding buffer, the refolded His-tagged IL-4 antagonist QY is eluted from the column with 100 mM EDTA and 10 mM Tris-HCl, pH 8.0. EDTA is used to chelate divalent cations that can potentially cause protein aggregation during dialysis. The His-tagged IL-4 antagonist QY is then dialyzed against 50 mM TrisHCl, pH 8.0, 5 mM CaCl₂, and 100 mM NaCl to remove EDTA. The protein is concentrated to approximately 2 mg/ml.

### Removal of the His-tag.

The His-tag is cleaved overnight by incubation of each mg fusion protein with 10 U thrombin (Sigma) in PBS buffer. The mix is incubated at room temperature overnight. The reaction can be stopped with 1 mM PMSF. Cleavage products are separated by chromatography. Residual thrombin can be removed with pAminonezamidine-Agarose (Sigma) either by batch or chromatographic methods.

The cleavage specificity is confirmed by N-terminus sequencing of blotted IL-4 antagonist QY. The His-tag is removed by passing the cleaved protein through Ni-fractogel beads. The purified samples are dialyzed in a desired buffer and kept at 4°C for short term storage or stored at -70°C in the presence of 25% glycerol.

*Analysis of correct refolding.*

The structural integrity of the murine IL-4 antagonist QY is analyzed in a cellular binding assay of murine IL-4 and the murine IL-4 antagonist QY to the murine IL-4 receptor. The functionality of murine IL-4 and the murine IL-4 antagonist QY is determined in a T cell proliferation assay (for details see Example 2).

### EXAMPLE 2: In vitro assays for quantitative determinations of murine IL-4 and the murine IL-4 antagonist QY

In vitro assays for murine IL-4 and the murine IL-4 antagonist QY include an ELISA-type assay and cellular assays. The ELISA-type assay provides information about the integrity of the murine Il-4 epitope recognized by the anti-murine IL-4 antibody. Information about the structural integrity of the murine IL-4 antagonist QY is obtained from a cellular binding assay analyzing binding of murine IL-4 and the murine IL-4 antagonist QY to the murine IL-4 receptor. The functionality of murine IL-4 and the murine IL-4 antagonist QY is determined in a T cell proliferation assay.

### ELISA analysis

The concentration of murine IL-4 or murine IL-4 mutants is determined by ELISA (Mouse IL-4 ELISA MAX™ Deluxe, BioLegend GmbH, Fell) according to the manufacturers instructions.

### Cellular binding assay for murine IL-4 and murine IL-4 antagonist QY

Binding of murine I1-4 or the murine IL-4 antagonist QY to IL-4 receptor molecules on the surface of murine CTLL-2 cells is detected by FACScan analysis using a biotinylated anti-IL-4 antibody and fluorescent-labeled streptavidin. Murine CTLL-2 cells are IL-2-dependent and typically express 2000-5000 I1-4 receptors per cell.

Murine CTLL-2 cells, obtained from ATCC (ATCC TIB 214), are cultured in RPMI 1640 containing 8% fetal calf serum, 100 units/ml penicillin and 100 µg/ml streptomycin, supplemented with 100 ng/ml IL-2. For the cellular binding assay 5 x 10⁶ cells are incubated for 30 min at 37°C in 150 µl of PBS (pH 7.4) containing 1% (w/v) BSA, 1 x 10⁻⁹ M recombinant murine (rmu) IL-4 or the murine IL-4 antagonist QY. The mixture is chilled to 4°C, washed once in a large volume of PBS (pH 7.4) containing 1% (w/v) BSA, resuspended in 150 *µ*l of PBS (pH 7.4) and subjected to FACScan analysis.

### T cell proliferation assay

I1-4 induced proliferation of CTLL-2 cells is determined by incorporation of [³H] thymidine as described (Duschl *et al*., 1992). The inhibitory effect of the murine IL-4 antagonist QY is determined by adding increasing amounts of the antagonist to IL-4.

CTLL-2 cells are incubated in 0.2 ml aliquots at a density of 5 x 10⁵/ml with log 2 dilutions of murine I1-4 for 3 days, before the amount of [³H] thymidine incorporated during the final 4 hr is determined. The concentration of IL-4 yielding half-maximal response is used for determining the inhibitory activity of the murine IL-4 antagonist QY. In these experiments, log 2 dilutions of the murine IL-4 antagonist QY are added to wild-type murine IL-4.

### EXAMPLE 3: Synthesis and characterization of thermogelling PLGA-PEG-PLGA hydrogels

The biodegradable triblock polymer described in this example has a PEG (1000)/PEG (1500) weight ratio of 20/80, a PLG/PEG weight ratio of 2.1 (68/32), and a lactide/glycolide molar ratio of 85/15. Synthesis of the triblock copolymer is performed according to published protocols (Quiao *et al*., 2005; WO 02/102309).

### Copolymer synthesis.

Polyethylene glycol (PEG 1000 and PEG 1500) is purchased from Fluka, poly(DL-lactide) from Sigma, glycolide (1,4-Dioxane-2,5-dione) from Sigma, and stannous 2-ethylhexanoate from Aldrich.

A total of 50 g of DL-lactide, glycolide and PEG are used for polymerization (28.9 g DL-lactide, 5.1 g glycolide, 3.2 g PEG 1000, 12.8 g PEG 1500). Under nitrogen atmosphere, PEG 1000 and PGE 1500 (weight ratio of 20/80) is dried in a threenecked flask (equipped with a nitrogen inlet, thermometer, and distillation head for removal of water) under vacuum and stirring at 120°C for 2 h. Stannous 2-ethylhexanoate (0.2% w/w) is added into a vigorously dried polymerization tube followed by the addition of DL-lactide and gycolide monomers. Then the tube is sealed under vacuum. The sealed tube was immersed and kept in an oil bath thermostated at 150°C. After 8 h the tube was cooled to room temperature, then broken and the product was dissolved in cold water. After completely dissolved, the copolymer solution is heated to 80°C to precipitate the copolymer and to remove the water-soluble lowmolecular weight copolymers and unreacted monomers. The supernatant is decanted, the precipitated copolymer is again dissolved in cold water followed by heating to induce precipitation. This process of dissolution followed by precipitation is repeated three times. Finally, the copolymer is dried under vacuum at room temperature until constant weight.

### Molecular weight determination.

The molecular weight of the copolymer is determined by gel permeation chromatography using polystyrene standards as described by Quiao *et al*. (2005).

### Measurement of gelation temperature.

The gelation temperature is determined as described by Quiao *et al.* (2005). A 20 ml transparent vial containing a 2.6 g heavy magnetic bar (10 x 5 mm i.d.) and 10 g water solution of the copolymer (15% w/w; and 25% w/w), is placed in a water bath. The solution is heated at a constant rate of 2°C per minute with constant stirring at 200 rpm. When the magnetic bar stops stirring due to gelation of the solution, the temperature read from the thermometer is determined as gelation temperature.

### EXAMPLE 4: In vitro degradation of thermogelling PLGA-PEG-PLGA hydrogels

The *in vitro* degradation behavior of the copolymer of Example 3 is evaluated by the mass loss and the molecular weight reduction with time upon incubation in phosphate buffered saline (PBS).

Samples (0.5 ml) are incubated in PBS pH 7.4 at 37°C under mild agitation in a water bath. The solid residues are removed from the incubation medium at scheduled time intervals and lyophilized. The samples are weighted and the weight loss is calculated. Then the solid residues are solved in cold water and analyzed by gel permeation chromatography using polystyrene standards as described by Quiao *et al.* (2005).

### EXAMPLE 5: In vitro release of the murine IL-4 antagonist QY from PLGA-PEG-PLGA/murine IL-4 antagonist QY composits

The PLGA-PEG-PLGA triblock copolymer of Example 3 is dissolved at room temperature in distilled water containing different concentrations of the murine IL-4 antagonist QY (0.5 mg/ml up to 2.0 mg/ml) to make a 15% w/w or 25% w/w solution. Then 1 ml of the formulation is placed in a 20 ml vial, incubated at 37°C for 2 min until gelling, and 10 ml of PBS pH 7.4 is added. The vial is shaken at 100 rpm at 37°C. At specified sample collection times, a sample is withdrawn and replaced by an identical volume of PBS pH 7.4 to maintain release conditions.

The amount of released murine IL-4 antagonist QY is determined by bicinchoninc acid (BCA) assay using BCA™ Protein Assay Kit (Pierce, USA) and by an ELISA-type assay as described in Example 2. The structural integrity of released murine IL-4 antagonist QY is determined by a cellular binding assay as described in Example 2. The functionality of released murine IL-4 antagonist QY is determined by inhibition of IL-4-induced T cell proliferation as described in Example 2.

### EXAMPLE 6: Biodegradation and release characteristics of PLGA-PEG-PLGA/murine IL-4 antagonist QY composits

To test the *in vivo* gel formation behavior and the release characteristics of composits of PLGA-PEG-PLGA hydrogels containing the murine IL-4 antagonist QY, the composits are injected subcutaneously into mice that have been anesthetized with ethyl ether. The resulting gel implants are then allowed to develop in vivo over the experimental period. At each of the post-injection sampling points, the mice are sacrificed and the gel implants are removed from the subcutaneous injection site. After determination of the volume of the removed gel implant, the remaining in vitro releasable amount of the murine IL-4 antagonist QY is determined as described Example 5.

Two groups of wild-type BALB/c mice (each group: 16 mice) are analyzed for the in vivo gel formation behavior and the release characteristics: group 1: 15% (w/w) polymer containing 200 µg murine IL-4 antagonist QY; group 2: 25% (w/w) polymer containing 200 µg murine IL-4 antagonist QY. The analysis is performed according to following schedule: implantation of 200 *µ*l polymer composit on day 0, analysis of size and residual release in two mice on days 1,2,3,4,5,10,20 and 30.

### EXAMPLE 7: Effect of co-injection of polymer-embedded IL-4 antagonist QY and alum-adsorbed ovalbumin on the humoral immune response in mice

This example shows the effect of subcutaneously injected polymer-embedded IL-4 antagonist QY followed by the injection of alum-adsorbed ovalbumin (OVA) at the site of the gelled polymer composit, on the humoral immune response and development of allergic symptoms in mice.

Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

### Preparation of alum-adsorbed ovalbumin (OVA).

OVA (100 µg) is solved in 0.3 ml of PBS, pH 7.4, and mixed with 0.70 ml Imject Alum.

### Treatment procedure.

Mice are injected subcutaneously 200 µl of the PLGA-PEG-PLGA triblock copolymer of Example 3 dissolved in distilled water containing increasing doses of the murine IL-4 antagonist QY (0.5 mg/ml, 1.0 mg/ml or 2.0 mg/ml). The final concentration of the copolymer in these experiments is 15% w/w or 25% w/w. A control group receives the copolymer without embedded IL-4 antagonist QY. Before immunization with OVA analyses of OVA-specific antibodies, cytokine levels in serum and FOXP3 and GATA3 mRNA expression are performed. Six hours later, 100 µl alum-adsorbed OVA (10 µg in 100 µl of PBS/Imject Alum) are injected subcutaneously at the site of the gelled polymer composit (50 µl at each side of the gelled polymer). At day 5 after immunization with OVA, one group of mice (group 6) are injected again 100 µl of the PLGA-PEG-PLGA triblock copolymer of Example 3 containing the murine IL-4 antagonist QY. At day 10 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Six groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer (100 µg antagonist), immunization with alum-OVA); group 4: one injection of loaded polymer (200 µg antagonist), immunization with alum-OVA; group 5: one injection of loaded polymer (400 µg antagonist), immunization with alum-OVA; and group 6: two injections of loaded polymer (total: 600 µg antagonist), immunization with alum-OVA.

### Analysis of serum levels of OVA-specific antibodies.

Mice are bled at day 0 before immunization with OVA and 10 days later. Murine anti-OVA IgE and IgG subclasses are determined by ELISA. Plates are coated with 10 µg OVA in 100 µl 0.1 M NaHCO₃ for 6 h at 37°C, followed by blocking with 200 µl 3% BSA in PBS, pH 7.4, for 2 h at 37°C. After washing, 100 µl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

### Analysis of serum levels of cytokines.

The cytokine supernatant is profiled using a panel of 27 cytokines including the Th2 cytokines IL-4, IL-5 and IL-13.

### Analysis of FOXP3 and GATA3 mRNA expression.

The read-out for T cell differentiation is FOXP3 and GATA3 mRNA expression, which are inversely regulated. In addition the release is followed in realtime using STAT6 responsive Luciferase systems reporter systems.

### Analysis of immediate cutaneous hypersensitivity.

Active cutaneous anaphylaxis is tested by skin test after i.v. injection of 200 µl of 0.5% Evans Blue dye in PBS, pH 7.4. Thereafter, the skin of the belly is shaved and four injection sites are marked with a felt tip pen on the skin. Two of the marked sites are injected intradermally with 50 µl PBS, pH 7.4, containing 50 µg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

### Analysis of anaphylactic shock symptoms.

Mice are injected i.v. 500 µg OVA in 200 µl 0.5% Evans Blue solution. After 15 min, symptoms of an anaphylactic shock are assessed including bluing (no - 0; slight - 1; strong = 2), pilo erection (no = 0; slight - 1; strong - 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

### Analysis of T cell phenotype in inguinal lymphnodes.

Right and left inguinal lymph nodes are removed and T cell phenotype is tested by *in vitro* re-stimulation with allergen (ELISPOT/Luminex).

### EXAMPLE 8: Specific immunotherapy of OVA-sensitized mice with alum-adsorbed OVA and PLGA-PEG-PLGA/murine IL-4 antagonist QY composits

This example shows the efficacy of immunotherapy in OVA-sensitized mice using subcutaneously injected polymer-embedded IL-4 antagonist QY and co-injected alum-adsorbed OVA at the site of the gelled polymer composit.

Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

*Preparation of alum-adsorbed ovalbumin* (OVA). OVA (100 µg) is solved in 0.3 ml of PBS, pH 7.4, and mixed with 0.70 ml Imject Alum (Pierce/KMF).

*Allergic sensitization procedure.* Mice are immunized three times by i.p. injection with 10 µg OVA in 200 µl of PBS, pH 7.4, mixed with 70 µl Imject Alum as adjuvant. The second i.p. injection is performed 7 days after the first injection and the third injection 14 days after the first injection.

*Immunotherapy.* One week after the third immunization with OVA, mice are subjected to two different modalities of immunotherapy, one based on conventional treatment with increasing doses of alum-adsorbed OVA, the other based on treatment with increasing doses of alum-adsorbed OVA in the presence of polymer-embedded murine IL-4 antagonist QY. Subcutaneous administration of increasing doses of alum-adsorbed OVA is performed at 10-day intervals. Subcutaneous administration of polymer-embedded murine IL-4 antagonist QY is performed at day 0 and day 5 within the 10-day intervals.

For immunotherapy of mice with alum-adsorbed OVA only, 100 µl of PBS/Imject Alum containing increasing doses of OVA (5, 15, 30, 60, 90 µg) are injected subcutaneously at 10-day intervals.

For immunotherapy of mice with alum-adsorbed OVA in the presence of polymer-embedded murine IL-4 antagonist QY, 200 µl of distilled water containing the PLGA-PEG-PLGA triblock copolymer of Example 3 (15% w/w or 25% w/w) and 400 µg of the murine IL-4 antagonist QY, are injected subcutaneously first. After gelling of the polymer composit, 100 µl of PBS/Imject Alum containing increasing doses of OVA (5, 15, 30, 60, 90 µg) are injected subcutaneously at the site of the gelled polymer composit (50 µl at each side of the gelled polymer). At day 5 within each 10-day interval, 100 µl of distilled water containing the PLGA-PEG-PLGA triblock copolymer of Example 3 (15% w/w or 25% w/w) and 200 µg of the murine IL-4 antagonist QY, are injected subcutaneously at the site of the first injection. The injection procedure is repeated three times at a different subcutaneous site until the highest dose of alum-adsorbed OVA is injected.

At day 10 after the last immunotherapeutic treatment, serum levels of OVA-specific antibodies are analyzed. Furthermore, immediate hypersensitivity in skin responses upon challenge via intradermal injection of OVA and the development of anaphylactic shock upon i.v. injection of OVA are assessed.

Three groups of wild-type BALB/c mice (each group: 10 mice) are analyzed: group 1: immunization (3 x alum-OVA), therapy with non-loaded polymer and PBS; group 2: immunization (3 x alum-OVA), therapy with loaded polymer and alum-OVA; and group 3: immunization (3 x alum-OVA), therapy with alum-OVA.

### Analysis of serum levels of OVA-specific antibodies.

Mice are bled at day 0 before immunization with OVA and 10 days later. Murine anti-OVA IgE and IgG subclasses are determined by ELISA. Plates are coated with 10 µg OVA in 100 µl 0.1 M NaHCO₃ for 6 h at 37°C, followed by blocking with 200 µl 3% BSA in PBS, pH 7.4, for 2 h at 37°C. After washing, 100 µl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

### Analysis of serum levels of cytokines.

The cytokine supernatant is profiled using a panel of 27 cytokines including the Th2 cytokines IL-4, IL-5 and IL-13.

### Analysis of FOXP3 and GATA3 mRNA expression.

The read-out for T cell differentiation is FOXP3 and GATA3 mRNA expression, which are inversely regulated. In addition the release is followed in realtime using STAT6 responsive Luciferase systems reporter systems.

### Analysis of immediate cutaneous hypersensitivity.

Active cutaneous anaphylaxis is tested by skin test after i.v. injection of 200 µl of 0.5% Evans Blue dye in PBS, pH 7.4. Thereafter, the skin of the belly is shaved and four injection sites are marked with a felt tip pen on the skin. Two of the marked sites are injected intradermally with 50 µl PBS, pH 7.4, containing 50 µg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

### Analysis of anaphylactic shock symptoms.

Mice are injected i.v. 500 µg OVA in 200 µl 0.5% Evans Blue solution. After 15 min, symptoms of an anaphylactic shock are assessed including bluing (no = 0; slight = 1; strong = 2), pilo erection (no = 0; slight - 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

### Analysis of T cell phenotype in inguinal lymphnodes.

Right and left inguinal lymph nodes are removed and T cell phenotype is tested by in vitro re-stimulation with allergen (ELISPOT/Luminex).

### EXAMPLE 9: Cloning, expression and purification of the human IL-4 antagonist RY

*Cloning of the human IL-4 antagonist RY (R121D*/*Y124D).*

The sequence of the native mature human IL-4 protein is modified to include a thrombin cleavable N-terminal 6xHis-tag and the QY (R121D/Y124D) mutations (see SEQID 01). The protein sequence is translated into DNA using codon optimization for expression in prokaryontic *E*. *coli* cells (Puigbo *et al*, 2007; Grote *et al,* 2005). The resulting sequence (SEQID 04) is synthesized and amplified with primers 1 (IL4his-for) and 3 (MOIL4mut-back) using PCR with Pfu polymerase according to the manufacture and annealing temperatures rising from initial 5 cycles at 50°C to 60°C and 30 cycles total. The resulting PCF fragment is gel-purified in 1% agarose (GeneJET Gel Extraction Kit, Fermentas) and transferred to a restriction enzyme double digest containing Nde *I* and *Xho I* (Fermentas). The restriction digest is again gel-purified and the fragment is ligated into *Nde I* and *Xho I* cut pET-22b (Novagen) expression vector.

### Expression.

Recombinant His-tagged murine IL-4 antagonist QY (R121D/Y124D) is expressed in *E*. *coli* (BL21(DE3)pLysS; Novagen) at 37°C. Cultures are grown in 1-L batches in minimal medium containing 100 µg/ml ampicillin and 34 µg/ml chloramphenicol. The minimal medium contains the following components: 0.1 M phosphate buffer, pH 7.0, 2 mM MgSO₄, 17 mM NaCl, 0.1 mM CaCl₂, 5 mg/L FeSO₄, 0.2 mg/L (NH₄)MO₇O₂₄, 1 mg/L H₃BO₃, 0.2 mg/L CoCl₂, 0.2 mg/L CuSO₄, 2 mg/L MnSO₄ and 2 mg/L ZnSO₄, supplemented with thymidine, biotin, folic acid, pantothenic acid, niacinamide, pyroxidine phosphate, and thiamine (1 mg/L each). The expression is induced by the addition of isopropyl-D-thiogalactopyranoside to a final concentration of 1 mM after the culture reaches an OD₆₀₀ of approximately 0.6. The cells are allowed to grow for 4h and then harvested by centrifugation at 5000 x g for 10 min at 4°C. The pellet is stored at -20°C.

### Refolding from inclusion bodies.

The insoluble His-tagged human IL-4 antagonist RY (R121D/Y124D) is refolded according to a published protocol (Razeghifard, 2004). The *E*. *coli* pellet containing the recombinant IL-4 antagonist RY is resuspended in 15 ml of 50 mM Tris-HCl, pH 8.0, and 5 mM EDTA (lysis buffer) containing 2 mg lysozyme and incubated at room temperature for 20 min. The cell lysate is diluted with 60 ml lysis buffer and sonicated. It is then centrifuged at 11,000 rpm for 30 min at 4°C to collect the pellet containing inclusion bodies. The pellet is washed twice with 60 ml 2 M urea in lysis buffer and collected by centrifugation as described above. Finally, the pellet is resuspended in 25 ml of 6 M GdnHCl, 50 mM potassium phosphate, pH 8.0, and 1 mM reduced glutathione (extraction buffer) and stirred at room temperature for 10 min. The solution is centrifuged to remove the insoluble portion.

The guanidine-extracted IL-4 antagonist RY is loaded onto 15 ml of Ni-charged HisTrap™ FF columns (GE Healthcare) equilibrated with the extraction buffer. The beads are washed with 8 column volumes of 7 M urea and 50 mM potassium phosphate, pH 6.8, to remove the cellular proteins. The His-tagged IL-4 antagonist QY is refolded on the column by the gradual removal of urea through a linear gradient expanding from 100% unfolding buffer to 100% refolding buffer (380 ml at a flow rate of 1 ml/min) using a fast protein liquid chromatography ÄKTA™ system (Pharmacia). The unfolding buffer is composed of 7 M urea, 100 mM NaCl, 50 mM potassium phosphate, pH 6.8, 1 mM reduced glutathione, and 1 mM oxidized glutathione; the refolding buffer has the same composition but without urea. After washing the column with 40 ml refolding buffer, the refolded His-tagged IL-4 antagonist QY is eluted from the column with 100 mM EDTA and 10 mM Tris-HCl, pH 8.0. EDTA is used to chelate divalent cations that can potentially cause protein aggregation during dialysis. The His-tagged IL-4 antagonist QY is then dialyzed against 50 mM Tris-HCl, pH 8.0, 5 mM CaCl₂, and 100 mM NaCl to remove EDTA. The protein is concentrated to approximately 2 mg/ml.

### Removal of the His-tag.

The His-tag is cleaved overnight by incubation of each mg fusion protein with 10 U thrombin (Sigma) in PBS buffer. The mix is incubated at room temperature overnight. The reaction is stopped with 1 mM PMSF. Cleavage products are separated by chromatography. Residual thrombin is removed with pAminonezamidine-Agarose (Sigma) either by batch or chromatographic methods.

The cleavage specificity is confirmed by N-terminus sequencing of blotted IL-4 antagonist RY. The His-tag is removed by passing the cleaved protein through Ni-fractogel beads. The purified samples are dialyzed in a desired buffer and kept at 4°C for short term storage or stored at -70°C in the presence of 25% glycerol.

### Analysis of correct refolding.

The structural integrity of the human IL-4 antagonist RY is analyzed in a cellular binding assay of human IL-4 and the human IL-4 antagonist RY to the human IL-4 receptor. The functionality of human IL-4 and the human IL-4 antagonist RY is determined in a T cell proliferation assay (for details see Example 10).

### EXAMPLE 10: In vitro assays for quantitative determinations of human IL-4 and the human IL-4 antagonist RY

In vitro assays for human IL-4 and the human IL-4 antagonist RY include an ELISA-type assay and cellular assays. The ELISA-type assay provides information about the integrity of the human IL-4 epitope recognized by the anti-human IL-4 antibody. Information about the structural integrity of the human IL-4 antagonist RY is obtained from a cellular binding assay analyzing binding of human IL-4 and the human IL-4 antagonist RY to the human IL-4 receptor. The functionality of human IL-4 and the human IL-4 antagonist RY is determined in a T cell proliferation assay.

### ELISA analysis

The concentration of human IL-4 or human IL-4 mutants is determined by ELISA (LEGEND MAX™ Human IL-4 ELISA Kit, BioLegend GmbH) according to the manufacturers instructions.

### Cellular binding assay

Binding of human I1-4 or the human IL-4 antagonist RY to IL-4 receptor molecules on the surface of human Raji B-lymphoma cells is detected by FACScan analysis using a biotinylated anti-IL-4 antibody and fluorescent-labeled streptavidin.

Human Raji B-lymphoma cells are obtained from ATCC (ATCC CLL-86) and cultured in RPMI 1640 containing 10% fetal calf serum. The cells (5 x 10⁶) are incubated for 30 min at 37°C in 150 *µ*l of PBS (pH 7.4) containing 1% (w/v) BSA, 1 x 10⁻⁹ M human IL-4 or the human IL-4 antagonist RY. The mixture is chilled to 4°C, washed once in a large volume of PBS (pH 7.4) containing 1% (w/v) BSA, resuspended in 150 *µ*l of PBS (pH 7.4) and subjected to FACScan analysis.

### T cell proliferation assay

Peripheral blood mononuclear cells are obtained from healthy donors, purified by Ficoll-Hypaque centrifugation (Pharmacia) and stored in aliquots at -80°C. Thawed cells are cultured for 7 days with 9 µg/ml phytohemagglutinine (PHA; HA-15, Wellcome) and comprise at this stage a high percentage of activated T-cells (PHA-blasts). Cells (10⁵/200 *µ*l) are incubated with log 2 dilutions of human IL-4 for three days, before the amount of [³H] thymidine incorporated during the final 4 hr is determined. The concentration of IL-4 yielding half-maximal response is used for determining the inhibitory activity of the human IL-4 antagonist RY. In these experiments, log 2 dilutions of the human IL-4 antagonist RY are added to wild-type human IL-4.

### EXAMPLE 11: In vitro release of the human IL-4 antagonist RY from PLGA-PEG-PLGA/human IL-4 antagonist RY composits

The PLGA-PEG-PLGA triblock copolymer of Example 3 is dissolved at room temperature in distilled water containing different concentrations of the human IL-4 (and IL-13) antagonist RY (0.5 mg/ml up to 2.0 mg/ml) to make a 15% w/w or 25% w/w solution. Then 1 ml of the formulation is placed in a 20 ml vial, incubated at 37°C for 2 min until gelling, and 10 ml of PBS pH 7.4 is added. The vial is shaken at 100 rpm at 37°C. At specified sample collection times, a sample is withdrawn and replaced by an identical volume of PBS pH 7.4 to maintain release conditions.

The amount of released human IL-4 antagonist RY is determined by bicinchoninc acid (BCA) assay using BCA™ Protein Assay Kit (Pierce, USA) and by an ELISA-type assay as described in Example 10. The structural integrity of released human IL-4 antagonist RY is determined by a cellular binding assay as described in Example 10. The functionality of released human IL-4 antagonist RY is determined by inhibition of IL-4-induced T cell proliferation as described in Example 10.

### SEQUENCES

### SEQID 01

### Human Interleukin-4, Pro-peptide sequence

Ref.: Yokota et al 1986, PNAS 83: 5894-5898; UniProtKB/Swiss-Prot: P05112-1
Genbank M13982.1, HUMIL4
>ENA|M13982|M13982.1 Human interleukin 4 (IL-4) mRNA, complete cds. : Location:1..1000

1 gatcgttagc ttctcctgat aaactaattg cctcacattg tcactgcaaa tcgacaccta
61 ttaatgggtc tcacctccca actgcttccc cctctgttct tcctgctagc atgtgccggc
121 aactttgtcc acggacacaa gtgcgatatc accttacagg agatcatcaa aactttgaac
181 agcctcacag agcagaagac tctgtgcacc gagttgaccg taacagacat ctttgctgcc
241 tccaagaaca caactgagaa ggaaaccttc tgcagggctg cgactgtgct ccggcagttc
301 tacagccacc atgagaagga cactcgctgc ctgggtgcga ctgcacagca gttccacagg
361 cacaagcagc tgatccgatt cctgaaacgg ctcgacagga acctctgggg cctggcgggc
421 ttgaattcct gtcctgtgaa ggaagccaac cagagtacgt tggaaaactt cttggaaagg
481 ctaaagacga tcatgagaga gaaatattca aagtgttcga gctgaatatt ttaatttatg
541 agtttttgat agctttattt tttaagtatt tatatattta taactcatca taaaataaag
601 tatatataga atct

### SEQID 02

Human Interleukin-4, Pro-peptide Translated DNA sequence Signal peptide sequence is underlined

### SEQID 03

Human Interleukin-4 (RY-Double mutant, N-terminal His-tagged mature peptide sequence

Ref.: Tony et al 1994, Eur J. Biochem 225 : 659-665 ; Razeghifard 2004, Prot Exp Pur 37 :180-186

His-Tag with thrombin cleavage site and mutated sites are underlined

MGSSHHHHHHSSGLVPRGSHMHKCDITLQEIIKTLNSLTEQKTLCTELTVTDIFAASKNTTEKETFCRAATVLR
QFYSHHEKDTRCLGATAQQFHRHKQLIRFLKRLDRNLWGLAGLNSCPVKEANQSTLENFLERLKTIMDEKDSKC
SS

### SEQID 04

Human Interleukin-4, N-terminal His-tagged mature peptide sequence, *E*. *coli* K12 optimized codon usage sequence

Ref.: Puigbo et al 2007, NAR 35:W126-W131; Grote et al 2005, NAR 33: W526-31.

ATGGGTTCTTCTCACCACCACCACCACCACTCTTCTGGTCTGGTTCCGCGTGGTTCTCACATGCACAAATGCGA
CATCACCCTGCAGGAAATCATCAAAACCCTGAACTCTCTGACCGAACAGAAAACCCTGTGCACCGAACTGACCG
TTACCGACATCTTCGCGGCGTCTAAAAACACCACCGAAAAAGAAACCTTCTGCCGTGCGGCGACCGTTCTGCGT
CAGTTCTACTCTCACCACGAAAAAGACACCCGTTGCCTGGGTGCGACCGCGCAGCAGTTCCACCGTCACAAACA
GCTGATCCGTTTCCTGAAACGTCTGGACCGTAACCTGTGGGGTCTGGCGGGTCTGAACTCTTGCCCGGTTAAAG
AAGCGAACCAGTCTACCCTGGAAAACTTCCTGGAACGTCTGAAAACCATCATGGACGAAAAAGACTCTAAATGC
TCTTCT

**SEQID 05**

Primer 1 (IL4his-for) for expression subcloning.

5'end of Human/Mouse IL-4 (RY/QY mutant) N-terminal His-tagged mature peptide. Contains Nde *I* restriction site for pET-22b+ (underlined)

IL4his-for, 29 bp

5' -AAAAAACCATATGGGTTCTTCTCACCACC

initial annealing temp: 54,5°C

overall annealing temp: 59,2°C

### SEQID 06

### Primer 2 (HUIL4mut-back) for expression subcloning.

3'end of Human IL-4 (RY mutant) N-terminal His-tagged mature peptide. Contains Stop codon (TAA) and Xho *I* restriction site for pET-22b+ (underlined). (Double digest of *Nde I* and *Xho I* is possible in e.g. NEB buffer 2)

HUIL4mut-back, 29 bp

5'-AAAACTCGAGTTAAGAAGAGCATTTAGAG

initial annealing temp: 41,3°C

overall annealing temp: 55,2°C

### SEQID 07

Murine Interleukin-4, Pro-peptide sequence

Ref.: Noma et al 1986, Nature 319: 640-646; UniProtKB/Swiss-Prot: P07750 Genbank X03532.1, Mouse mRNA for IgG1 induction factor >ENA|X03532|x03532.1 Mouse mRNA for IgG1 induction factor : Location:1..1000

1 atttgttagc atctcttgat aaacttaatt gtctctcgtc actgacggca cagagctatt
61 gatgggtctc aacccccagc tagttgtcat cctgctcttc tttctcgaat gtaccaggag
121 ccatatccac ggatgcgaca aaaatcactt gagagagatc atcggcattt tgaacgaggt
181 cacaggagaa gggacgccat gcacggagat ggatgtgcca aacgtcctca cagcaacgaa
241 gaacaccaca gagagtgagc tcgtctgtag ggcttccaag gtgcttcgca tattttattt
301 aaaacatggg aaaactccat gcttgaagaa gaactctagt gttctcatgg agctgcagag
361 actctttcgg gcttttcgat gcctggattc atcgataagc tgcaccatga atgagtccaa
421 gtccacatca ctgaaagact tcctggaaag cctaaagagc atcatgcaaa tggattactc
481 gtagtactga gccaccatgc tttaacttat gaatttttaa tggttttatt tttaatattt
541 atatatttat aattcataaa ataaaatatt tgtataatgt

### SEQID 08

Murine Interleukin-4, Pro-peptide Translated DNA sequence. Signal peptide sequence is underlined

MGLNPQLVVILLFFLECTRSHIHGCDKNHLREIIGILNEVTGEGTPCTEMDVPNVLTATKNTTESELVCRASKVL
RIFYLKHGKTPCLKKNSSVLMELQRLFRAFRCLDSSISCTMNESKSTSLKDFLESLKSIMQMDYS

### SEQID 09

Murine Interleukin-4, QY-Double mutant, His-tagged. N-terminal His-tagged mature peptide sequence,His-Tag with thrombin cleavage site and mutated sites are underlined.

Ref.: Grunewald et al 1997 JBC 272: 1480-1483

MGSSHHHHHHSSGLVPRGSHMHIHGCDKNHLREIIGILNEVTGEGTPCTEMDVPNVLTATKNTTESELVCRASKV
LRIFYLKHGKTPCLKKNSSVLMELQRLFRAFRCLDSSISCTMNESKSTSLKDFLESLKSIMDMDDS

### SEQID 10

Murine Interleukin-4 QY-Double mutant, His-tagged N-terminal His-tagged mature peptide sequence, *E. coli* K12 optimized codon usage sequence

Ref.: Puigbo et al 2007, NAR 35:W126-W131; Grote et al 2005, NAR 33: W526-31.

ATGGGTTCTTCTCACCACCACCACCACCACTCTTCTGGTCTGGTTCCGCGTGGTTCTCACATGCACATCCACGG
TTGCGACAAAAACCACCTGCGTGAAATCATCGGTATCCTGAACGAAGTTACCGGTGAAGGTACCCCGTGCACCG
AAATGGACGTTCCGAACGTTCTGACCGCGACCAAAAACACCACCGAATCTGAACTGGTTTGCCGTGCGTCTAAA
GTTCTGCGTATCTTCTACCTGAAACACGGTAAAACCCCGTGCCTGAAAAAAAACTCTTCTGTTCTGATGGAACT
GCAGCGTCTGTTCCGTGCGTTCCGTTGCCTGGACTCTTCTATCTCTTGCACCATGAACGAATCTAAATCTACCT
CTCTGAAAGACTTCCTGGAATCTCTGAAATCTATCATGGACATGGACGACTCT

### SEQID 11

### Primer 3 (MOIL4mut-back) for subcloning.

3'end of Murine IL-4 (QY mutant) N-terminal His-tagged mature peptide. Contains Stop codon (TAA) and *Xho I* restriction site for pET-22b+ (underlined)

MOIL4mut-back, 29 bp

5'-AAAACTCGAGTTAAGAGTCGTCCATGTCC

initial annealing temp: 50,5 C

overall annealing temp: 59,8 C

## Claims

1. Pharmaceutical composition made of one or more preparations and comprising a physiologically effective dose of at least one IL-4 and/or IL-13 inhibitor and at least one allergene, and a matrix, wherein at least the inhibitor is solved or embedded, or whereon at least the inhibitor is coated or adsorbed, wherein the matrix is selected as to enable prolonged release of the inhibitor.

2. Composition according to claim 1, wherein the inhibitor is selected from the group consisting of antagonistic IL-4 and/or IL-13 derivatives, soluble cytokine receptor constructs with specificity for IL-4 and/or IL-13 and/or precursors thereof, monoclonal antibodies specific for IL-4 and/or IL-13 and/or their precursors and/or their receptors, binding fragments of such antibodies, proteinaceous constructs with specificity for IL-4 and/or IL-13 and/or their precursors and/or their receptors, and aptamers with specificity for IL-4 and/or IL-13 and/or their precursors and/or their receptors.

3. Composition according to claim 1 or 2, wherein at least two different inhibitors are comprised, wherein at least one inhibitor inhibits IL-4 and at least one other inhibitor inhibits IL-13, or wherein at least one inhibitor is comprised, which inhibits both, IL-4 and IL-13.

4. Composition according to one of the claims 1 to 3,
wherein the inhibitor has a plasma half-life of less than 7 days, preferably less than 1 day, most preferably less than 6 or 5 hours.

5. Composition according to one of the claims 1 to 4,
wherein the inhibitor is a human IL-4 mutant with one to three mutations preferably in at least one of the positions R121, Y124 and S125.

6. Composition according to one of the claims 1 to 5,
wherein the matrix is a biodegradable or biostable organic polymer, preferably biodegradable, more preferably thermogelling, in particular selected from the group consisting of styrene-isobutylene based block copolymer, olefin polymer, polyethylene, polypropylene, polyethylene oxide (PEO), polypropylene oxide (PPO), polyvinyl chloride, polytetrafluoroethylene, fluorinated ethylene propylene copolymer, polyvinyl acetate, polystyrene, poly (ethylene teraphthalate), polyurethane, polyurea, silicone rubbers, polyamides, polycarbonates, polyaldehydes, natural rubbers, polyester copolymers, styrene-butadiene copolymers ethylene vinyl acetate, polyorthoesters, polyiminocarbonates, aliphatic polycarbonates, polycaprolactone (PCL), poly-D,L-lactic acid (PDLLA), poly-L-lactic acid (PLLA), lactides of said lactic acids, polyphosphazenes polyethylene oxide, polyethylene teraphtholate (PET), polybutylene teraphtholate (PBT), PEBAX, Nylon, polyorthoesters, polylactic acids, polyglycolic acids, albumin, monomethoxypoly(ethylene glycol) (MPEG) or copolymers or mixtures of any of the above including poly(lactic-coglycolic acid) (PLGA), copolymers of L-lactide and D,L-lactide, diblock copolymers consisting of MPEG and PCL, MPEG and (PCL-ran-PLLA), MPEG and PLGA, triblock copolymers consisting of PLGA-PEG-PLGA, PEG-PLGA-PEG, PEG-PCL-PEG, diblock and triblockpolymers consisting of PEO and PLLA, Poloxamers.

7. Composition according to claim 6, wherein the polymer is thermogelling and wherein the gelling temperature above which gelling commences is between 20 °C and 40 °C, preferably between 25 °C and 30 °C.

8. Composition according to one of the claims 6 or 7,
wherein the 90 weight-% degradation of the polymer in body environment and/or 90 weight-% release of the inhibitor from the polymer is completed within 1 to 10 days, preferably within 1 to 2 days.

9. Composition according to one of the claims 1 to 8,
wherein additionally an adjuvans is comprised, whereein tha adjuvans is preferably selected from the group consisting of liposomes, aluminum salts, Montanide emulsions, granulocyte-macrophage colony stimulating factor (GM-CSF), adjuvans based on inulin, virosomes and polyphosphazenes, most preferably is selected from aluminum phosphate or aluminum hydroxide gels.

10. Composition according to one of the claims 1 to 5,
wherein the matrix is an adjuvans according to claim 9.

11. Composition according to one of the claims 1 to 10,
wherein:
all components are mixed as a single preparation, or
the matrix and the inhibitor are mixed as a first preparation and the allergen and adjuvans are mixed as a second preparation.

12. Composition according to one of the claims 1 to 11,
wherein the composition is galenically prepared for administration by subcutaneous injection.

13. Use of a composition according to one of the claims 1 to 12 for the preparation of a medicine for the treatment of immunological disorders, in particular allergic reactions, preferably wherein the medicine is administered in a therapeutically effective dose to a person in need of treatment of a immunological disorder.

14. Method for manufacturing a pharmaceutical composition according to one of the claims 12, wherein the components are mixed with each other in an physiologically effective amount to obtain a composition according to claim 11, wherein optionally galenic compounds are additionally admixed to the preparation.

15. Method for treatment of an immunological disorder, in particular an allergic reaction, wherein a composition according to one of the claims 1 to 12 is administered in an therapeutically effective dose to a person in need of the treatment.


SEQUENCE LISTING

<110> PLS-Design GmbH
Grunwald, Thomas
Schmidt-Weber, Carsten B.

<120> Pharmaceutical Composition for Treatment of Allergic Reactions

<130> notyetallotted

<160> 11

<170> PatentIn version 3.3

<210> 1
<211> 614
<212> DNA
<213> Human

<400> 1
gatcgttagc ttctcctgat aaactaattg cctcacattg tcactgcaaa tcgacaccta 60

ttaatgggtc tcacctccca actgcttccc cctctgttct tcctgctagc atgtgccggc 120

aactttgtcc acggacacaa gtgcgatatc accttacagg agatcatcaa aactttgaac 180

agcctcacag agcagaagac tctgtgcacc gagttgaccg taacagacat ctttgctgcc 240

tccaagaaca caactgagaa ggaaaccttc tgcagggctg cgactgtgct ccggcagttc 300

tacagccacc atgagaagga cactcgctgc ctgggtgcga ctgcacagca gttccacagg 360

cacaagcagc tgatccgatt cctgaaacgg ctcgacagga acctctgggg cctggcgggc 420

ttgaattcct gtcctgtgaa ggaagccaac cagagtacgt tggaaaactt cttggaaagg 480

ctaaagacga tcatgagaga gaaatattca aagtgttcga gctgaatatt ttaatttatg 540

agtttttgat agctttattt tttaagtatt tatatattta taactcatca taaaataaag 600

tatatataga atct 614

<210> 2
<211> 153
<212> PRT
<213> Human

<400> 2

<210>  3
<211>  150
<212>  PRT
<213>  Artificial

<220>
<223>  mutant of human IL-4

<400>  3

Met Gly Ser Ser His His His His His His Ser Ser Gly Leu Val Pro
1               5                   10                  15

Arg Gly Ser His Met His Lys Cys Asp Ile Thr Leu Gln Glu Ile Ile
            20                  25                  30

Lys Thr Leu Asn Ser Leu Thr Glu Gln Lys Thr Leu Cys Thr Glu Leu
        35                  40                  45

Thr Val Thr Asp Ile Phe Ala Ala Ser Lys Asn Thr Thr Glu Lys Glu
    50                  55                  60

Thr Phe Cys Arg Ala Ala Thr Val Leu Arg Gln Phe Tyr Ser His His
65                  70                  75                  80

Glu Lys Asp Thr Arg Cys Leu Gly Ala Thr Ala Gln Gln Phe His Arg
                85                  90                  95

His Lys Gln Leu Ile Arg Phe Leu Lys Arg Leu Asp Arg Asn Leu Trp
            100                 105                 110

Gly Leu Ala Gly Leu Asn Ser Cys Pro Val Lys Glu Ala Asn Gln Ser
        115                 120                 125

Thr Leu Glu Asn Phe Leu Glu Arg Leu Lys Thr Ile Met Asp Glu Lys
    130                 135                 140

Asp Ser Lys Cys Ser Ser
145                 150

<210>  4
<211>  450
<212>  DNA
<213>  Artificial

<220>
<223>  Human IL-4, tagged

<400>  4
atgggttctt ctcaccacca ccaccaccac tcttctggtc tggttccgcg tggttctcac      60

atgcacaaat gcgacatcac cctgcaggaa atcatcaaaa ccctgaactc tctgaccgaa     120

cagaaaaccc tgtgcaccga actgaccgtt accgacatct tcgcggcgtc taaaaacacc     180

accgaaaaag aaaccttctg ccgtgcggcg accgttctgc gtcagttcta ctctcaccac     240

gaaaaagaca cccgttgcct gggtgcgacc gcgcagcagt tccaccgtca caaacagctg     300

atccgtttcc tgaaacgtct ggaccgtaac ctgtggggtc tggcgggtct gaactcttgc     360

ccggttaaag aagcgaacca gtctaccctg gaaaacttcc tggaacgtct gaaaaccatc     420

atggacgaaa aagactctaa atgctcttct                                      450

<210>  5
<211>  29
<212>  DNA
<213>  Artificial

<220>
<223>  primer

<400>  5
aaaaaaccat atgggttctt ctcaccacc                                        29

<210>  6
<211>  29

<212>  DNA
<213>  Artificial

<220>
<223>  primer

<400>  6
aaaactcgag ttaagaagag catttagag                                          29


<210>  7
<211>  580
<212>  DNA
<213>  Murine

<400>  7
atttgttagc atctcttgat aaacttaatt gtctctcgtc actgacggca cagagctatt      60

gatgggtctc aacccccagc tagttgtcat cctgctcttc tttctcgaat gtaccaggag     120

ccatatccac ggatgcgaca aaaatcactt gagagagatc atcggcattt tgaacgaggt     180

cacaggagaa gggacgccat gcacggagat ggatgtgcca aacgtcctca cagcaacgaa     240

gaacaccaca gagagtgagc tcgtctgtag ggcttccaag gtgcttcgca tattttattt     300

aaaacatggg aaaactccat gcttgaagaa gaactctagt gttctcatgg agctgcagag     360

actctttcgg gcttttcgat gcctggattc atcgataagc tgcaccatga atgagtccaa     420

gtccacatca ctgaaagact tcctggaaag cctaaagagc atcatgcaaa tggattactc     480

gtagtactga gccaccatgc tttaacttat gaatttttaa tggttttatt tttaatattt     540

atatatttat aattcataaa ataaaatatt tgtataatgt                           580


<210>  8
<211>  140
<212>  PRT
<213>  Murine

<400>  8

Met Gly Leu Asn Pro Gln Leu Val Val Ile Leu Leu Phe Phe Leu Glu
1               5                   10                  15


Cys Thr Arg Ser His Ile His Gly Cys Asp Lys Asn His Leu Arg Glu
            20                  25                  30


Ile Ile Gly Ile Leu Asn Glu Val Thr Gly Glu Gly Thr Pro Cys Thr
        35                  40                  45


Glu Met Asp Val Pro Asn Val Leu Thr Ala Thr Lys Asn Thr Thr Glu
    50                  55                  60

Ser Glu Leu Val Cys Arg Ala Ser Lys Val Leu Arg Ile Phe Tyr Leu
65                  70                  75                  80

Lys His Gly Lys Thr Pro Cys Leu Lys Lys Asn Ser Ser Val Leu Met
                85                  90                  95

Glu Leu Gln Arg Leu Phe Arg Ala Phe Arg Cys Leu Asp Ser Ser Ile
            100                 105                 110

Ser Cys Thr Met Asn Glu Ser Lys Ser Thr Ser Leu Lys Asp Phe Leu
        115                 120                 125

Glu Ser Leu Lys Ser Ile Met Gln Met Asp Tyr Ser
    130                 135                 140

<210>  9
<211>  141
<212>  PRT
<213>  Artificial

<220>
<223>  Murine IL-4 mutant

<400>  9

Met Gly Ser Ser His His His His His His Ser Ser Gly Leu Val Pro
1               5                   10                  15

Arg Gly Ser His Met His Ile His Gly Cys Asp Lys Asn His Leu Arg
            20                  25                  30

Glu Ile Ile Gly Ile Leu Asn Glu Val Thr Gly Glu Gly Thr Pro Cys
        35                  40                  45

Thr Glu Met Asp Val Pro Asn Val Leu Thr Ala Thr Lys Asn Thr Thr
    50                  55                  60

Glu Ser Glu Leu Val Cys Arg Ala Ser Lys Val Leu Arg Ile Phe Tyr
65                  70                  75                  80

Leu Lys His Gly Lys Thr Pro Cys Leu Lys Lys Asn Ser Ser Val Leu
                85                  90                  95

Met Glu Leu Gln Arg Leu Phe Arg Ala Phe Arg Cys Leu Asp Ser Ser
            100                 105                 110

Ile Ser Cys Thr Met Asn Glu Ser Lys Ser Thr Ser Leu Lys Asp Phe
        115                 120                 125

Leu Glu Ser Leu Lys Ser Ile Met Asp Met Asp Asp Ser
    130                 135                 140

<210>  10
<211>  423
<212>  DNA
<213>  Artificial

<220>
<223>  Murine IL-4 mutant

<400>  10
atgggttctt ctcaccacca ccaccaccac tcttctggtc tggttccgcg tggttctcac      60

atgcacatcc acggttgcga caaaaaccac ctgcgtgaaa tcatcggtat cctgaacgaa     120

gttaccggtg aaggtacccc gtgcaccgaa atggacgttc cgaacgttct gaccgcgacc     180

aaaaacacca ccgaatctga actggtttgc cgtgcgtcta aagttctgcg tatcttctac     240

ctgaaacacg gtaaaacccc gtgcctgaaa aaaaactctt ctgttctgat ggaactgcag     300

cgtctgttcc gtgcgttccg ttgcctggac tcttctatct cttgcaccat gaacgaatct     360

aaatctacct ctctgaaaga cttcctggaa tctctgaaat ctatcatgga catggacgac     420

tct                                                                   423

<210>  11
<211>  29
<212>  DNA
<213>  Artificial

<220>
<223>  primer

<400>  11
aaaactcgag ttaagagtcg tccatgtcc                                        29